# EUROPÄISCHE PATENTSCHRIFT

(11) **EP 2 176 325 B1**
(45) Veröffentlichungstag und Bekanntmachung des Hinweises auf die Patenterteilung: **08.12.2021**
(21) Anmeldenummer: 08802558.0
(22) Anmeldetag: 24.09.2008
(51) Int. Cl.: C08J 3/12

(54) **SUPERABSORBIERENDE ZUSAMMENSETZUNG MIT TANNINEN ZUR GERUCHSKONTROLLE**
SUPER-ABSORBENT COMPOSITION COMPRISING TANNINS FOR ODOR CONTROL
COMPOSITION SUPERABSORBANTE CONTENANT DES TANNINS POUR LE CONTRÔLE DES ODEURS

(30) Priorität: 24.09.2007 DE 102007045724
(43) Veröffentlichungstag der Anmeldung: 21.04.2010
(73) Patentinhaber: Evonik Operations GmbH, 45128 Essen (DE)
(72) Erfinder: FURNO, Franck, 40545 Düsseldorf (DE); WATTEBLED, Laurent, 40589 Düsseldorf (DE); IX-MUND, Anne, 47647 Kerken (DE); SCHMIDT, Harald, 45481 Mülheim an der Ruhr (DE); HENN, Markus, 45894 Gelsenkirchen (DE); HARREN, Jörg, 47807 Krefeld (DE)
(74) Vertreter: Evonik Patent Association
(86) Internationale Anmeldenummer: PCT/EP2008/008081
(87) Internationale Veröffentlichungsnummer: WO 2009/040106

(56) Entgegenhaltungen:
- EP-A1- 1 510 562
- WO-A1-01/30748
- WO-A1-2010/052182
- US-A- 5 045 322

## Beschreibung

Die vorliegende Erfindung betrifft wasserabsorbierende Zusammensetzungen, ein Verfahren zur Herstellung einer wasserabsorbierenden Zusammensetzung, die durch dieses Verfahren erhältliche, wasserabsorbierende Zusammensetzungund die Verwendung einer wasserabsorbierenden Zusammensetzung.

Superabsorber sind wasserunlösliche, vernetzte Polymere, die in der Lage sind, unter Quellung und Ausbildung von Hydrogelen große Mengen an wässrigen Flüssigkeiten, insbesondere Körperflüssigkeiten, vorzugsweise Urin oder Blut, aufzunehmen und unter Druck zurückzuhalten. Im Allgemeinen betragen diese Flüssigkeitsaufnahmen das mindestens 10-Fache oder gar das mindestens 100-Fache des Trockengewichts der Superabsorber bzw. der superabsorbierenden Zusammensetzungen an Wasser. Durch diese charakteristischen Eigenschaften finden diese Polymere hauptsächlich Anwendung in Sanitärartikeln wie Babywindeln, Inkontinenzprodukten oder Damenbinden. Einen umfassenden Überblick über Superabsorber bzw. superabsorbierende Zusammensetzungen, ihre Anwendung und ihre Herstellung geben F. L. Buchholz und A. T. Graham (Herausgeber) in "Modern Superabsorbent Polymer Technology", Wiley-VCH, New York, 1998.

Die Herstellung der Superabsorber erfolgt in der Regel durch die radikalische Polymerisation säuregruppen-tragender, meist teilneutralisierter Monomere in Gegenwart von Vernetzern. Dabei lassen sich durch die Auswahl der Monomerzusammensetzung, der Vernetzer sowie der Polymerisationsbedingungen und der Verarbeitungsbedingungen für das nach der Polymerisation erhaltene Hydrogel Polymere mit unterschiedlichen Absorbereigenschaften herstellen. Weitere Möglichkeiten bietet die Herstellung von Pfropfpolymerisaten, beispielsweise unter Verwendung chemisch modifizierter Stärke, Cellulose und Polyvinylalkohol nach DE-OS 26 12 846.

DE 40 20 780 C1 offenbart die Nachbehandlung superabsorbierender Polymere durch Nachvernetzung der Oberflächen der Polymerteilchen. Durch die Nachvernetzung der Oberfläche der wasserabsorbierenden Polymerteilchen wird insbesondere das Absorptionsvermögen der Polymerteilchen unter der Einwirkung von Drücken erhöht.

DE 199 09 653 A1 und DE 199 09 838 A1 beschreiben pulverförmige, an der Oberfläche nachvernetzte, Wasser, wässrige oder seröse Flüssigkeiten oder Blut absorbierende Polymerisate, welche auf säuregruppen-tragenden Monomeren basieren und welche mit einem Oberflächennachvernetzungsmittel und einem Kation in wässriger Lösung beschichtet und nachvernetzt worden sind. Die in diesem Stand der Technik offenbarten Polymerisate weisen gegenüber herkömmlichen Polymerisaten vorteilhafte Absorptionseigenschaften, insbesondere eine hohe Permeabilität auf.

Beim längeren Tragen von absorbierende Polymere beinhaltenden Hygieneartikeln, insbesondere wenn diese bereits zu einem Teil Körperflüssigkeiten wie Urin aufgenommen haben, kann es bedingt durch die organischen Bestandteile des Urins und die Körperwärme der Trägerperson alsbald zu einer unangenehmen Geruchsbelastung kommen. Um dieser zu begegnen, wurden zahlreiche Versuche unternommen, durch entsprechende Beigaben in den vom Superabsorber verschiedenen Bestandteilen des Hygieneartikels eine Bindung der geruchsbildenden Stoffe zu erzielen oder den Geruch durch Parfums oder dergleichen zu übertönen. Das Einbringen von derartigen Substanzen in der Form von von Superabsorbern verschiedenen Bestandteilen wirkt sich oftmals negativ auf die Performance dieser Hygieneartikel während des Tragens aus. So werden durch die Körperflüssigkeiten oftmals die anfänglich vom Superabsorberbereich räumlich getrennt vorliegenden geruchshemmenden oder geruchsvermindernden Substanzen beispielsweise durch Hineinschwemmen in Superabsorber beinhaltende Bereich eines Hygieneartikels eingetragen, wo sie dann eine negative Auswirkung auf die Performance des Superabsorbers und damit des Hygieneartikels insgesamt zeigen. Ferner ist an diesem Konzept nachteilig, dass der Großteil der in den Hygieneartikel abgegebenen Körperflüssigkeit sich ohnehin in dem Superabsorber befindet und daher die außerhalb des Superabsorbers befindlichen geruchshemmenden oder geruchsvermindernden Substanzen ihre Wirkung schlechter entfalten können.

DE 198 25 486 und DE 199 39 662 A1 offenbaren die Kombination von Superabsorbern mit Cyclodextrin zur Geruchsverminderung. Diesem durchaus viel versprechenden Ansatz ist jedoch zu entnehmen, dass das Cyclodextrin nur unter bestimmten Bedingungen, nämlich wenn sichergestellt ist, dass das Cyclodextrin nicht von dem Superabsorber wieder separiert, seine geruchshemmende Wirkung in dem Superabsorber zeigt. Hierbei ist es bevorzugt, dass das Cyclodextrin zumindest in die Oberfläche des Superabsorberartikels eingearbeitet ist, indem Cyclodextrin und/oder Cyclodextrinderivate kovalent und/oder ionisch gebunden und/oder darin eingeschlossen sind.

Aus DE 103 34 271 sind weiterhin Superabsorberagglomerate bekannt, die eine Vielzahl von Geruchsbindern homogen in dem Agglomerat aufweisen können. Diese eine hervorragende Lösung für die Verwendung von Superabsorberfeinteilchen offenbarende Schrift stellt jedoch keine für die Anwendung in Hygieneartikeln besonders gut geeignete Superabsorber mit Geruchsbindungseigenschaften zur Verfügung. So sind neben einem effizienten und wirkungsvollen Einsatz der Geruchsbinder auch die durch diesen Geruchsbinder beeinflussten Superabsorbereigenschaften noch verbesserungswürdig.

DE-A-10 2005 055 497 lehrt, superabsorbierende Polymere dadurch das in Kontakt bringen mit Metallsalzen der Ricinolsäure und/oder mit Aminosäuren mit verbesserten geruchsbindenden Eigenschaften zu versehen.

EP-A1-1 510 562 beschreibt vernetzte Acrylat-Zusammensetzungen mit einem geruchshemmenden Wirkstoff aus Tee. In Beispiel 2 wurde als (halb)fermentierter Tee-Extrakt 0,5 Massenanteile Oolong-Tee-Extrakt mit 100 Massenanteilen des aus Referenzbeispiel 1 gewonnenen wasserabsorbierenden Harz vermischt.

Im Allgemeinen lag der vorliegenden Erfindung die Aufgabe zugrunde, die sich aus dem Stand der Technik ergebenden Nachteile abzumildern oder gar zu überwinden.

Eine erfindungsgemäße Aufgabe bestand darin, eine wasserabsorbierende Zusammensetzung bereitzustellen, die zum einen über gute Geruchsbindungseigenschaften verfügt. Dabei soll zum anderen gewährleistet sein, dass die Performance des Hygieneartikels, der diese geruchsbindende Superabsorberzusammensetzung beinhaltet, im wesentlichen gleich gut oder gar besser ist als die Performance des Hygieneartikels mit einem Superabsorber, der nicht wie die geruchsbindende superabsorbierende Zusammensetzung den Geruchsbinder beinhaltet. Insbesondere sollte die Performance-Eigenschaften der wasserabsorbierenden Zusammensetzung durch die Verwendung geruchsbindender Additive, welche in möglichst geringen Mengen eingesetzt werden sollen, möglichst gar nicht oder allenfalls geringfügig beeinflusst werden. Vorteilhafterweise sollten die Performance-Eigenschaften der wasserabsorbierenden Zusammensetzung durch den Zusatz des geruchsbindenden Additivs sogar verbessert werden.

Weiterhin sollte die wasserabsorbierende Zusammensetzung bei einem Kontakt mit wässrigen Körperflüssigkeiten, insbesondere mit Urin oder eisenhaltigen Flüssigkeiten, wie beispielsweise Blut oder Menstruationsflüssigkeit, möglichst nicht zu starken Verfärbungen neigen.

Ferner bestand eine erfindungsgemäße Aufgabe darin, ein Verfahren aufzuzeigen, mit dem eine solche wasserabsorbierende Zusammensetzung erhalten werden kann.

Zudem bestand eine erfindungsgemäße Aufgabe darin, einen Hygieneartikel bereitzustellen, der neben guten geruchsbindenden Eigenschaften auch eine gute Performance zeigt.

Außerdem lag der vorliegenden Erfindung als Aufgabe zugrunde, wasserabsorbierende Zusammensetzungen bereitzustellen, die sich allgemein in Verbunde einarbeiten lassen oder auch als Verbund oder als solche Verwendung in chemischen Produkten oder deren Bestandteilen finden können.

Einen Beitrag zur Lösung der eingangs genannten Aufgaben leistet eine wasserabsorbierende Zusammensetzung, beinhaltend wasserabsorbierende Polymergebilde, auf deren Oberfläche sich mindestens eine Tanninfraktion befindet, welche ein gemäß der hierin beschriebenen Testmethode bestimmtes Zahlenmittel des Molekulargewichtes von mindestens 1.000 g/mol, noch mehr bevorzugt von mindestens 1.100 g/mol und am meisten bevorzugt von mindestens 1.200 g/mol besitzt, wobei vorzugsweise ein Zahlenmittel des Molekulargewichtes von 10.000 g/mol, besonders bevorzugt von 5.000 g/mol nicht überschritten wird. Beispielsweise liegt das Zahlenmittel des Molekulargewichtes in einem Bereich von 1.250 bis 1.500 g/mol. In diesem Zusammenhang ist es insbesondere vorteilhaft, wenn die Tanninfraktion eine gemäß der hierin beschriebenen Testmethode bestimmte Polydispersität, definiert als M_{w}/Mₙ, wobei M_{w} das Gewichtsmittel des Molekulargewichtes und Mₙ das Zahlenmittel des Molekulargewichtes ist, von weniger als 5, besonders bevorzugt von weniger als 4 und am meisten bevorzugt von weniger als 3 aufweist.

Es ist erfindungsgemäß, wenn die wasserabsorbierende Zusammensetzung das Tannin bzw. die Tanninfraktion in einer Menge in einem Bereich von 0,001 bis 10 Gew.-%, jeweils bezogen auf das Gesamtgewicht der wasserabsorbierenden Polymergebilde, beinhaltet.

Einen Beitrag zur Lösung der eingangs genannten Aufgaben leistet auch eine wasserabsorbierende Zusammensetzung, beinhaltend wasserabsorbierende Polymergebilde, auf deren Oberfläche sich mindestens eine Tanninfraktion befindet, welche ein gemäß der hierin beschriebenen Testmethode bestimmtes Gewichtsmittel des Molekulargewichtes von mindestens 1.000 g/mol, noch mehr bevorzugt von mindestens 1.100 g/mol und am meisten bevorzugt von mindestens 1.200 g/mol besitzt, wobei vorzugsweise ein Gewichtsmittel des Molekulargewichtes von 10.000 g/mol, besonders bevorzugt von 5.000 g/mol nicht überschritten wird. Beispielsweise liegt das Gewichtsmittel des Molekulargewichtes in einem Bereich von 1.250 bis 1.500 g/mol. Auch in diesem Fall ist es insbesondere vorteilhaft, wenn die Tanninfraktion eine gemäß der hierin beschriebenen Testmethode bestimmte Polydispersität von weniger als 5, besonders bevorzugt von weniger als 4 und am meisten bevorzugt von weniger als 3 aufweist.

Es ist erfindungsgemäß, wenn die wasserabsorbierende Zusammensetzung das Tannin bzw. die Tanninfraktion in einer Menge in einem Bereich von 0,001 bis 10 Gew.-%, jeweils bezogen auf das Gesamtgewicht der wasserabsorbierenden Polymergebilde, beinhaltet.

Unter einem *"Tannin"* im Sinne der vorliegenden Erfindung werden im Allgemeinen natürlich vorkommende Polyphenole verstanden. Grundsätzlich können erfindungsgemäß sogenannte *"kondensierte Tannine"* oder aber *"hydrolysierbare Tannine"* eingesetzt werden, wobei der Einsatz hydrolysierbarer Tannine besonders bevorzugt und der Einsatz hydrolysierbarer Gallotannine am meisten bevorzugt ist.

Unter *"kondensierten Tanninen"* werden vorzugsweise Tannine verstanden, welche Oligomere oder Polymere aus Flavonoid-Einheiten darstellen, welche über C-C-Bindungen miteinander verknüpft sind. Solche kondensierten Tannine umfassen üblicherweise 2 bis 50 Flavonoid-Einheiten auf, können aber auch aus mehr als 50 Flavonoid-Einheiten bestehen. Als Flavonoid-Einheiten kommen dabei insbesondere Catechin und Epicatechin in Frage.

Unter *"hydrolysierbaren Tanninen"* werden vorzugsweise Tannine verstanden, die aus einem Polyol, beispielsweise einem Kohlenhydrat, als Kern bestehen, wobei an die OH-Gruppen dieses Kernmoleküls Gallsäure über Esterbindungen angebunden sind. Solche auf Gallsäure basierenden, hydrolysierbaren Tannine werden daher häufig auch als *"Gallotannine"* bezeichnet. Neben Gallsäure können die hydrolysierbaren Tannine aber auch auf Ellaginsäure basieren. Solche hydrolysierbaren Tannine werden häufig auch als *"Ellagitannine"* bezeichnet. Eine ausgezeichnete Übersicht über Tannine, insbesondere auch über hydroylsierbare Tannine und deren Charakterisierung bietet die Übersicht *"Tannin Chemistry"* der Autorin Ann E. Hagermann.

In diesem Zusammenhang insbesondere bevorzugt sind Tannine, welche sich von dem Grundgerüst der nachfolgenden Struktur I (β-1,2,3,4,6-Pentagalloyl-O-D-Glukose) 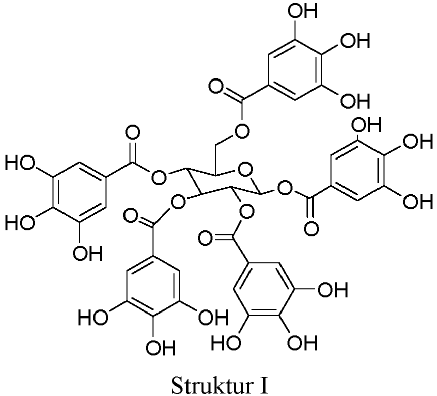 ableiten, wobei an mindestens eine der OH-Gruppen mindestens eines der an den Kohlenhydrat-Kern gebundenen Gallsäure-Moleküle (= Gallsäure-Molekül der ersten Ebene, gebunden als Galloyl-Ester), vorzugsweise an eine der OH-Gruppen in meta- oder in para-Position, vorzugsweise jedoch an mindestens eine der OH-Gruppen von 2, 3, 4 oder 5 der an den Kohlenhydrat-Kern gebundenen Gallsäure-Moleküle, mindestens ein weiteres Gallsäure-Molekül über eine Esterbindung (Digalloyl-Ester) gebunden ist (= Gallsäure-Molekül der zweiten Ebene). In diesem Zusammenhang ist es insbesondere bevorzugt, dass es sich bei dem Tannin um ein Tannin handelt, welches sich von dem Grundgerüst der Struktur I ableitet und welches mindestens ein, vorzugsweise zwei, drei, vier oder fünf Gallsäure-Moleküle der zweiten Ebene umfasst. Insbesondere jedoch ist es in diesem Zusammenhang bevorzugt, dass vorzugsweise mindestens 30 Gew.-%, besonders bevorzugt mindestens 50 Gew.-%, noch mehr bevorzugt mindestens 75 Gew.-% und am meisten bevorzugt mindestens 95 Gew.-% der in der wasserabsorbierenden Zusammensetzung enthaltenen Tannine ein Molekulargewicht von mehr als 940 g/mol, besonders bevorzugt mehr als 1.100 g/mol und darüber hinaus bevorzugt mehr als 1.200 g/mol aufweisen, wobei auch hier vorzugsweise ein Molekulargewicht von 10.000 g/mol, besonders bevorzugt von 5.000 g/mol nicht überschritten wird. Um festzustellen, ob ein gegebenes Tannin die vorstehend genannten Mengen an Tanninen mit einem Molekulargewicht von mehr als 940 g/mol aufweisen, kann es sich beispielsweise anbieten, dass entsprechende Tannin einer HPLC-Analyse zu unterwerfen und das dabei erhaltene Elugramm mit dem Elugramm von reiner β-1,2,3,4,6-Pentagalloyl-O-D-Glukose zu vergleichen. Ein solches Verfahren zur Bestimmung des Molekulargewichtes von Gallotanninen ist im Methodenteil der Beschreibung beschrieben. Neben β-1,2,3,4,6-Pentagalloyl-O-D-Glukose seien als weitere, erfindungsgemäß geeignete Tannine 2-6-di-O-galloyl-1,5-anhydro-D-glucitol (auch "Aceritannin" genannt), Eugeniin, Casuricitin, Corilagin, Geraniin, Davidiin, Casuariin, Casuarinin, Castalagin, Castlin, Stachyurin, Vescalagin, Vescalin, Euphorbin A, Oenothein B, Woodfordin C, Cuphiin D₁ und Eugeniflorin D₁ genannt.

Weiterhin können auch Tannine eingesetzt werden, bei denen eine oder mehrere freie OH-Gruppen durch Phosphat-Gruppen ersetzt worden sind, wie dies beispielsweise bei dem Produkt Floctan^{®} 4 von der Firma S. A. Ajinomoto OmniChem n. V., Belgien, der Fall ist.

Erfindungsgemäß besonders bevorzugt ist der Einsatz hydroylsierbarer Gallotannine mit den vorstehend genannten Gewichtsmitteln bzw. Zahlenmitteln des Molekulargewichtes, insbesondere auch mit den vorstehend genannten Polydispersitäten. Weiterhin ist es gemäß einer besonderen Ausführungsform der erfindungsgemäßen Zusammensetzung bevorzugt, dass die Gesamtheit aller in der erfindungsgemäßen, wasserabsorbierenden Zusammensetzung enthaltenen Tannine das eingangs genannte Gewichtsmittel bzw. das eingangs genannte Zahlenmittel des Molekulargewichtes aufweist.

Grundsätzlich können die in der erfindungsgemäßen Zusammensetzung enthaltenen Tannine, vorzugsweise die in der erfindungsgemäßen Zusammensetzung enthaltenen, hydrolysierbaren Gallotannine aus den unterschiedlichsten Pflanzlichen Quellen isoliert oder gegebenenfalls auch von kommerziellen Anbietern in bereits aufgereinigter Form bezogen werden. Die Isolierung von Tanninen aus pflanzlichen Extrakten, insbesondere auch die Isolierung von hydrolysierbaren Tanninen, ist dem Fachmann beispielsweise aus der vorstehend genannten Übersicht von Ann E. Hagermann oder aus Salminen et al., "Distribution of Hydrolysable Tannins in the Foliage of Finnisch Birch Species", Zeitschrift für Naturforschung, Vol. 57c, Seiten 248-256 (2002) bekannt. Als Beispiele für geeignete Quellen von erfindungsgemäß einsetzbaren Tanninen, insbesondere von erfindungsgemäß einsetzbaren, hydrolysierbaren Tanninen seien insbesondere Pflanzenextrakte ausgewählt aus der Gruppe bestehend aus *Rhus Semialata*-Extrakt*, Rhus Coriaria-*Extrakt, *Quercus Infectoria*-Extrakt*, Quercus Aegilops*-Extrakt*, Caesalpina Spinosa*-Extrakt, *Caesalpina D. Gyna*-Extrakt, *Valonea*-Extrakt, wie etwa *Quercus Valonea*-Extrakt oder *Quercus Macrolepsis*-Extrakt, Kastanienholz-Extrakt, wie etwa *Castanea Sativa*-Extrakt, und *Terminalia Chebula*-Extrakt oder Mischungen aus mindestens zwei davon, genannt, wobei Pflanzenextrakte aus *Rhus Semialata* und aus *Quercus Infectoria* bevorzugt und Pflanzenextrakte aus *Rhus Semialata* besonders bevorzugt sind.

Gegebenenfalls sind die aus pflanzlichen Quellen gewonnenen Tannine, insbesondere die aus pflanzlichen Quellen gewonnenen, hydrolysierbaren Tannine durch geeignete Trennverfahren, beispielsweise durch chromatographische Trennverfahren derart aufzuarbeiten, dass Tanninfraktionen erhalten werden, welche die eingangs genannten Werte für das bevorzugte Zahlenmittel bzw. für das bevorzugte Gewichtsmittel des Molekulargewichtes aufweisen.

Neben der Isolierung der erfindungsgemäß einsetzbaren Tanninfraktion aus pflanzlichen Quellen können geeignete Tanninfraktionen auch von kommerziellen Anbietern erworben werden. In Betracht kommen hier, neben dem bereit vorstehend genannten Produkt Floctan^{®} 4 beispielsweise auch die Produkte Tanal O2C, Floctan^{®} 1 oder Brewtan^{®} der Firma S. A. Ajinomoto OmniChem n. V., Belgien.

Weiterhin ist es gemäß einer besonders bevorzugten Ausführungsform der erfindungsgemäßen Zusammensetzung bevorzugt, dass sich die mindestens eine Tanninfraktion zumindest teilweise in Form von Partikeln auf der Oberfläche der wasserabsorbierenden Polymergebilde befindet, wobei es insbesondere bevorzugt ist, dass mindestens 30 Gew.-%, besonders bevorzugt mindestens 50 Gew.-%, noch mehr bevorzugt mindestens 75 Gew.-%, darüber hinaus bevorzugt mindestens 95 Gew.-% und am meisten bevorzugt 100 Gew.-% der Partikel der mindestens einen Tanninfraktion eine Größe in einem Bereich von 10 nm bis 300 µm, besonders bevorzugt 50 nm bis 50 µm und am meisten bevorzugt 100 nm bis 10 µm besitzen.

Es ist erfindungsgemäß, wenn die wasserabsorbierende Zusammensetzung das Tannin bzw. die Tanninfraktion in einer Menge in einem Bereich von 0,001 bis 10 Gew.-%, jeweils bezogen auf das Gesamtgewicht der wasserabsorbierenden Polymergebilde, beinhaltet. Es ist bevorzugt, wenn die wasserabsorbierende Zusammensetzung das Tannin bzw. die Tanninfraktion in einer Menge in einem Bereich von 0,1 bis 5 Gew.-% und am meisten bevorzugt in einer Menge in einem Bereich von 0,2 bis 2 Gew.-%, jeweils bezogen auf das Gesamtgewicht der wasserabsorbierenden Polymergebilde, beinhaltet. Besonders vorteilhaft im Zusammenhang mit der Farbstabilität der wasserabsorbierenden Zusammensetzung nach einem Kontakt mit Körperflüssigkeiten ist es jedoch, wenn die wasserabsorbierende Zusammensetzung das Tannin bzw. die Tanninfraktion in einer Menge von höchstens 0,75 Gew.-%, besonders bevorzugt höchstens 0,5 Gew.-% und am meisten bevorzugt höchstens 0,4 Gew.-%, jeweils bezogen auf das Gesamtgewicht der wasserabsorbierenden Polymergebilde, beinhaltet. Gemäß einer besonderen Ausführungsform der erfindungsgemäßen, wasserabsorbierenden Zusammensetzung beinhaltet diese das Tannin bzw. die Tanninfraktion in einer Menge von 0,2 bis 0,4 Gew.-%, bezogen auf das Gesamtgewicht der wasserabsorbierenden Polymergebilde.

Gegebenfalls können die Partikel der Tanninfraktion über einen Binder auf der Oberfläche der wasserabsorbierenden Polymergebilde immobilisiert sein, wobei als Binder grundsätzlich organische oder anorganische Binder eingesetzt werden können. Als Binder kommen dabei insbesondere thermoplastische Schmelzklebstoffe, hydrophile, vorzugsweise wasserlösliche Polymere oder auch Wasser selbst in Betracht. Als thermoplastische Schmelzklebstoffe können dabei insbesondere diejenigen partikulären Verbindungen eingesetzt werden, die in der WO-A-2005/011860 als thermoplastische Klebstoffe genannt sind. Als hydrophile, vorzugsweise wasserlösliche Polymere können insbesondere Polyvinylalkohole, die durch teilweises oder vollständiges Verseifen von Polyvinylacetat erhalten werden können, oder Polyalkylenglykole, insbesondere Polyethylenglykole mit einem Molekulargewicht in einem Bereich von 1.000 bis 50.000 g/mol, besonders bevorzugt in einem Bereich von 1.500 bis 25.000 g/mol und am meisten bevorzugt in einem Bereich von 2.000 bis 15.000 g/mol eingesetzt werden.

Weiterhin hat es sich als vorteilhaft erwiesen, dass sich auf der Oberfläche der in der erfindungsgemäßen, wasserabsorbierende Zusammensetzung enthaltenen wasserabsorbierende Polymergebilde nicht nur die vorstehend genannte Tanninfraktion, besonders bevorzugt die vorstehend beschriebenen hydrolysierbaren Tannine mit den vorstehend beschriebenen Eigenschaften, sondern zusätzlich hierzu auch ein oder mehrere Komplexbildner befinden. Es hat sich nämlich gezeigt, dass durch den kombinierten Einsatz von hydrolysierbaren Tanninen und Komplexbildnern nicht nur die geruchsbindenden Eigenschaften weiter deutlich verbessert werden können, sondern dass zudem auch die Farbstabilität der wasserabsorbierenden Zusammensetzung bei einem Kontakt mit Körperflüssigkeiten deutlich verbessert werden kann. Auch ist es durch den kombinierten Einsatz von hydrolysierbaren Tanninen und Komplexbildnern möglich, die Tannine in geringeren Mengen einzusetzen, was die Farbstabilität zusätzlich verbessert.

Einen Beitrag zur Lösung der eingangs genannten Aufgaben leistet daher auch eine wasserabsorbierende Zusammensetzung, beinhaltend wasserabsorbierende Polymergebilde, auf deren Oberfläche sich ein oder mehrere Komplexbildner sowie mindestens eine Tanninfraktion befinden, wobei die Tanninfraktion ein gemäß der hierin beschriebenen Testmethode bestimmtes Zahlenmittel des Molekulargewichtes von mindestens 1.000 g/mol, noch mehr bevorzugt von mindestens 1.100 g/mol und am meisten bevorzugt von mindestens 1.200 g/mol besitzt, wobei vorzugsweise ein Zahlenmittel des Molekulargewichtes von 10.000 g/mol, besonders bevorzugt von 5.000 g/mol nicht überschritten wird. Beispielsweise liegt das Zahlenmittel des Molekulargewichtes in einem Bereich von 1.250 bis 1.500 g/mol. In diesem Zusammenhang ist es insbesondere vorteilhaft, wenn die Tanninfraktion eine gemäß der hierin beschriebenen Testmethode bestimmte Polydispersität, definiert als M_{w}/Mₙ, wobei M_{w} das Gewichtsmittel des Molekulargewichtes und Mₙ das Zahlenmittel des Molekulargewichtes ist, von weniger als 5, besonders bevorzugt von weniger als 4 und am meisten bevorzugt von weniger als 3 aufweist.

Einen Beitrag zur Lösung der eingangs genannten Aufgaben leistet auch eine wasserabsorbierende Zusammensetzung, beinhaltend wasserabsorbierende Polymergebilde, auf deren Oberfläche sich ein oder mehrere Komplexbildner sowie mindestens eine Tanninfraktion befindet, wobei die Tanninfraktion ein gemäß der hierin beschriebenen Testmethode bestimmtes Gewichtsmittel des Molekulargewichtes von mindestens 1.000 g/mol, noch mehr bevorzugt von mindestens 1.100 g/mol und am meisten bevorzugt von mindestens 1.200 g/mol besitzt, wobei vorzugsweise ein Gewichtsmittel des Molekulargewichtes von 10.000 g/mol, besonders bevorzugt von 5.000 g/mol nicht überschritten wird. Beispielsweise liegt das Gewichtsmittel des Molekulargewichtes in einem Bereich von 1.250 bis 1.500 g/mol. Auch in diesem Fall ist es insbesondere vorteilhaft, wenn die Tanninfraktion eine gemäß der hierin beschriebenen Testmethode bestimmte Polydispersität von weniger als 5, besonders bevorzugt von weniger als 4 und am meisten bevorzugt von weniger als 3 aufweist.

Einen Beitrag zur Lösung der eingangs genannten Aufgaben leistet auch eine wasserabsorbierende Zusammensetzung, beinhaltend wasserabsorbierende Polymergebilde, auf deren Oberfläche sich ein oder mehrere Komplexbildner sowie mindestens eine Tanninfraktion beinhaltend Tannine befindet, wobei es sich bei dieser Tanninfraktion um die vorstehend genannten Tanninfraktionen handelt (Tanninfraktion mit einem gemäß der hierin beschriebenen Testmethode bestimmten Gewichtsmittel des Molekulargewichtes von mindestens 1.000 g/mol, Tanninfraktion mit einem gemäß der hierin beschriebenen Testmethode bestimmten Zahlenmittel des Molekulargewichtes von mindestens 1.000 g/mol oder Tanninfraktion beinhaltend hydrolysierbare Gallotannine), wobei in diesem Falle die Tanninfraktionen in aufgereinigter Form oder aber in Form von Pflanzenextrakten, insbesondere Tee-Extrakten, in der erfindungsgemäßen absorbierenden Zusammensetzung enthalten sein können.

Unter "Komplexbildnern" werden vorzugsweise Verbindungen verstanden, die über zwei oder mehr funktionelle Gruppen verfügen, welche an mehrwertige Metall-Kationen koordinieren können. Bei diesen funktionellen Gruppen handelt es sich beispielsweise um organische Säuregruppen, Amin- oder Imingruppen oder Sulfonsäuregruppen. Erfindungsgemäß besonders bevorzugte Komplexbildner sind Aminopolycarbonsäuren, also Aminocarbonsäuren mit drei oder mehr Carboxylgruppen oder deren Salze. Unter diesen Aminopolycarbonsäuren insbesondere bevorzugt sind Diethylentriaminpentaacetat, Triethylentetraaminhexaacetat, Cyclohexan-1,2-diamintetraacetat, N-2-Hydroxyethylethylendiamintriacetat, Ethylenglykoldiethyletherdiamintetraacetat, Ethylendiamintetrapropionat, N-Alkyl-N' -carboxymethylaspartat, N-Alkenyl-N' -carboxymethylaspartat, S,S-Ethylen-diamin-N,N'-dibernsteinsäure und deren Alkalimetallsalze, Erdalkalimetallsalze, Ammoniumsalze und Aminsalze. Von diesen sind Diethylentriaminpentaacetat, N-2-Hydroxyethylethylendiamintriacetat sowie S,S-Ethylendiamin-N,N'-dibern-steinsäure und deren Salze besonders bevorzugt und das Pentanatriumsalz des Diethylentriaminpentaacetat am meisten bevorzugt.

Im Zusammenhang mit den vorstehend beschriebenen Komplexbildnern ist es weiterhin bevorzugt, dass die wasserabsorbierende Zusammensetzung den Komplexbildner in einer Menge in einem Bereich von 0,001 bis 5 Gew.-%, besonders bevorzugt in einem Bereich von 0,01 bis 1 Gew.-% und am meisten bevorzugt in einer Menge in einem Bereich von 0,05 bis 0,5 Gew.-%, bezogen auf das Gesamtgewicht der wasserabsorbierenden Polymergebilde, beinhaltet.

Die in der erfindungsgemäßen Zusammensetzung enthaltenen, wasserabsorbierenden Polymergebilde basieren vorzugsweise auf teilneutralisierten, vernetzten Polyacrylaten.

Dabei sind erfindungsgemäß bevorzugte wasserabsorbierende Polymergebilde insbesondere Fasern, Schäume oder Teilchen, wobei Fasern und Teilchen besonders bevorzugt und Teilchen am meisten bevorzugt sind.

Erfindungsgemäß bevorzugte Polymerfasern sind so dimensioniert, dass sie in oder als Garne für Textilien und auch direkt in Textilien eingearbeitet werden können. Es ist erfindungsgemäß bevorzugt, dass die Polymerfasern eine Länge im Bereich von 1 bis 500 mm, bevorzugt 2 bis 500 mm und besonders bevorzugt 5 bis 100 mm und einen Durchmesser im Bereich von 1 bis 200 Denier, bevorzugt 3 bis 100 Denier und besonders bevorzugt 5 bis 60 Denier besitzen.

Erfindungsgemäß bevorzugte Polymerteilchen sind so dimensioniert, dass sie eine mittlere Teilchengröße gemäß ERT 420.2-02 im Bereich von 10 bis 3000 µm, vorzugsweise 20 bis 2000 µm und besonders bevorzugt 150 bis 850 µm oder 150 bis 600 µm aufweisen. Dabei ist es insbesondere bevorzugt, dass der Anteil der Polymerteilchen mit einer Partikelgröße in einem Bereich von 300 bis 600 µm mindestens 30 Gew.-%, besonders bevorzugt mindestens 40 Gew.-%, darüber hinaus bevorzugt mindestens 50 Gew.-% und am meisten bevorzugt mindestens 75 Gew.-%, bezogen auf das Gesamtgewicht der wasserabsorbierenden Polymerteilchen, beträgt. Gemäß einer anderen Ausführungsform der erfindungsgemäßen wasserabsorbierenden Polymergebilde beträgt der Anteil der Polymerteilchen mit einer Partikelgröße in einem Bereich von 150 bis 850 µm mindestens 50 Gew.-%, besonders bevorzugt mindestens 75 Gew.-%, darüber hinaus bevorzugt mindestens 90 Gew.-% und am meisten bevorzugt mindestens 95 Gew.-%, bezogen auf das Gesamtgewicht der wasserabsorbierenden Polymerteilchen.

In einer bevorzugten Ausführungsform der erfindungsgemäßen wasserabsorbierenden Polymergebilde basieren diese auf
(α1) 20-99,999 Gew.-%, bevorzugt 55-98,99 Gew.-% und besonders bevorzugt 70-98,79 Gew.-% polymerisierten, ethylenisch ungesättigten, säuregruppen-tragenden Monomeren oder deren Salze oder polymerisierten, ethylenisch ungesättigten, einen protonierten oder quarternierten Stickstoff beinhaltenden Monomeren, oder deren Mischungen, wobei mindestens ethylenisch ungesättigte, säuregruppenhaltige Monomere, vorzugsweise Acrylsäure, beinhaltende Mischungen besonders bevorzugt sind,
(a2) 0-80 Gew.-%, vorzugsweise 0-44,99 Gew.-% und besonders bevorzugt 0,1-44,89 Gew.-% polymerisierten, monoethylenisch ungesättigten, mit (α1) copolymerisierbaren Monomeren,
(a3) 0,001-5 Gew.-%, vorzugsweise 0,01-3 Gew.-% und besonders bevorzugt 0,01-2,5 Gew.-% eines oder mehrerer Vernetzer,
(a4) 0-30 Gew.-%, vorzugsweise 0-5 Gew.-% und besonders bevorzugt 0,1-5 Gew.-% eines wasserlöslichen Polymeren,
(a5) 0-20 Gew.-%, vorzugsweise 2,5-15 Gew.-% und besonders bevorzugt 5-10 Gew.-% Wasser, sowie
(a6) 0-20 Gew.-%, vorzugsweise 0-10 Gew.-% und besonders bevorzugt 0,1-8 Gew.-% eines oder mehrerer Hilfsmittel, wobei die Summe der Gewichtsmengen (α1) bis (a6) 100 Gew.-% beträgt.

Die monoethylenisch ungesättigten, säuregruppenhaltigen Monomere (α1) können teilweise oder vollständig, bevorzugt teilweise neutralisiert sein. Vorzugsweise sind die monoethylenisch ungesättigten, säuregruppenhaltigen Monomere zu mindestens 25 Mol-%, besonders bevorzugt zu mindestens 50 Mol-% und darüber hinaus bevorzugt zu 50-80 Mol-% neutralisiert. Gemäß besondere Ausführungsformen der erfindungsgemäßen Zusammensetzung beinhaltet dieses jedoch wasserabsorbierende Polymergebilde, welche zu höchstens 78 Mol-%, besonders bevorzugt zu höchstens 70 Mol-% und zu mindestens 50 Mol-% neutralisiert sind. In diesem Zusammenhang kann gemäß einer Ausgestaltung der erfindungsgemäßen Zusammensetzung der Neutralisationsgrad der wasserabsorbierenden Polymergebilde in einem Bereich von 65 bis 80 Mol-%, besonders bevorzugt in einem Bereich von 70 bis 78 Mol-% und gemäß einer anderen Ausgestaltung in einem Bereich von 45 bis 65 Mol-%, besonders bevorzugt in einem Bereich von 50 bis 60 Mol-% liegen. Weiterhin wird auf DE 195 29 348 A1 verwiesen, deren Offenbarung hiermit als Referenz eingeführt wird. Die Neutralisation kann teilweise oder ganz auch nach der Polymerisation erfolgen. Ferner kann die Neutralisation mit Alkalimetallhydroxiden, Erdalkalimetallhydroxiden, Ammoniak sowie Carbonaten und Bicarbonaten erfolgen. Daneben ist jede weitere Base denkbar, die mit der Säure ein wasserlösliches Salz bildet. Auch eine Mischneutralisation mit verschiedenen Basen ist denkbar. Bevorzugt ist die Neutralisation mit Ammoniak und Alkalimetallhydroxiden, besonders bevorzugt mit Natriumhydroxid und mit Ammoniak.

Ferner können bei einem Polymer die freien Säuregruppen überwiegen, so dass dieses Polymer einen im sauren Bereich liegenden pH-Wert aufweist. Dieses saure wasserabsorbierende Polymer kann durch ein Polymer mit freien basischen Gruppen, vorzugsweise Amingruppen, das im Vergleich zu dem sauren Polymer basisch ist, mindestens teilweise neutralisiert werden. Diese Polymere werden in der Literatur als "Mixed-Bed Ion-Exchange Absorbent Polymers" (MBIEA-Polymere) bezeichnet und sind unter anderem in der WO 99/34843 A1 offenbart. Die Offenbarung der WO 99/34843 A1 wird hiermit als Referenz eingeführt und gilt somit als Teil der Offenbarung. In der Regel stellen MBIEA-Polymere eine Zusammensetzung dar, die zum einen basische Polymere, die in der Lage sind, Anionen auszutauschen, und andererseits ein im Vergleich zu dem basischen Polymer saures Polymer, das in der Lage ist, Kationen auszutauschen, beinhalten. Das basische Polymer weist basische Gruppen auf und wird typischerweise durch die Polymerisation von Monomeren erhalten, die basische Gruppen oder Gruppen tragen, die in basische Gruppen umgewandelt werden können. Bei diesen Monomeren handelt es sich vor allen Dingen um solche, die primäre, sekundäre oder tertiäre Amine oder die entsprechenden Phosphine oder mindestens zwei der vorstehenden funktionellen Gruppen aufweisen. Zu dieser Gruppe von Monomeren gehören insbesondere Ethylenamin, Allylamin, Diallylamin, 4-Aminobuten, Alkyloxycycline, Vinylformamid, 5-Aminopenten, Carbodiimid, Formaldacin, Melamin und dergleichen, sowie deren sekundäre oder tertiäre Aminderivate.

Bevorzugte ethylenisch ungesättigte, säuregruppenhaltige Monomere (α1) sind vorzugsweise diejenigen Verbindungen, die in der WO 2004/037903 A2 als ethylenisch ungesättigte, säuregruppenhaltige Monomere (α1) genannt werden. Besonders bevorzugte ethylenisch ungesättigte, säuregruppenhaltige Monomere (α1) sind Acrylsäure und Methacrylsäure, wobei Acrylsäure am meisten bevorzugt ist.

Gemäß einer Ausführungsform des erfindungsgemäßen Verfahrens werden wasserabsorbierende Polymergebilde eingesetzt, bei denen die monoethylenisch ungesättigten, mit (α1) copolymerisierbare Monomere (α2) Acrylamide, Methacrylamide oder Vinylamide sind.

Bevorzugte (Meth)acrylamide sind neben Acrylamid und Methacrylamid alkylsubstituierte (Meth)acrylamide oder aminoalkylsubstituierte Derivate des (Meth)acrylamids, wie N-Methylol(meth)acrylamid, N,N-Dimethylamino-(meth)acrylamid, Dimethyl(meth)acrylamid oder Diethyl(meth)acrylamid. Mögliche Vinylamide sind beispielsweise N-Vinylamide, N-Vinylformamide, N-Vinylacetamide, N-Vinyl-N-Methylacetamide, N-Vinyl-N-methylformamide, Vinylpyrrolidon. Unter diesen Monomeren besonders bevorzugt ist Acrylamid.

Gemäß einer anderen Ausführungsform des erfindungsgemäßen Verfahrens werden wasserabsorbierende Polymergebilde eingesetzt, bei denen die monoethylenisch ungesättigten, mit (α1) copolymerisierbare Monomere (α2) wasserlösliche Monomere sind. In diesem Zusammenhang sind insbesondere Alkoxypolyalkylenoxid(meth)acrylate wie Methoxypolyethylenglykol(meth)acrylate bevorzugt.

Des Weiteren sind als monoethylenisch ungesättigte, mit (α1) copolymerisierbaren Monomere (α2) in Wasser dispergierbare Monomere bevorzugt. Als in Wasser dispergierbare Monomere sind Acrylsäureester und Methacrylsäureester, wie Methyl(meth)acrylat, Ethyl(meth)acrylat, Propyl(meth)acrylat oder Butyl(meth)-acrylat bevorzugt.

Die monoethylenisch ungesättigten, mit (α1) copolymerisierbaren Monomere (α2) umfassen weiterhin Methylpolyethylenglykolallylether, Vinylacetat, Styrol und Isobutylen.

Als Vernetzer (α3) werden vorzugsweise diejenigen Verbindungen eingesetzt, die in der WO 2004/037903 A2 als Vernetzer (α3) genannt werden. Unter diesen Vernetzern sind wasserlösliche Vernetzer besonders bevorzugt. Am meisten bevorzugt sind dabei N,N'-Methylenbisacrylamid, Polyethylenglykoldi(meth)acrylate, Triallylmethylammoniumchlorid, Tetraallylammoniumchlorid sowie mit 9 Mol Ethylenoxid pro Mol Acrylsäure hergestelltes Allylnonaethylenglykolacrylat besonders bevorzugt.

Als wasserlösliche Polymere (α4) können in den Polymergebilden wasserlösliche Polymerisate, wie teil- oder vollverseifter Polyvinylalkohol, Polyvinylpyrrolidon, Stärke oder Stärkederivate, Polyglykole oder Polyacrylsäure enthalten, vorzugsweise einpolymerisiert sein. Das Molekulargewicht dieser Polymere ist unkritisch, solange sie wasserlöslich sind. Bevorzugte wasserlösliche Polymere sind Stärke oder Stärkederivate oder Polyvinylalkohol. Die wasserlöslichen Polymere, vorzugsweise synthetische wie Polyvinylalkohol, können auch als Pfropfgrundlage für die zu polymerisierenden Monomeren dienen.

Als Hilfsmittel (α6) sind vorzugsweise Stellmittel, Geruchsbinder, oberflächenaktive Mittel oder Antioxidatien sowie diejenigen Additive, die zur Herstellung der Polymergebilde eingesetzt wurden (Initiatoren usw.) in den Polymergebilden enthalten.

In einer besonderen Ausführungsform der erfindungsgemäßen Polymergebilde basieren diese zu mindestens 50 Gew.-%, vorzugsweise zu mindestens 70 Gew.-% und darüber hinaus bevorzugt zu mindestens 90 Gew.-% auf carboxylatgruppentragenden Monomeren. Es ist erfindungsgemäß weiterhin bevorzugt, dass die Komponente (α1) zu mindestens 50 Gew.-% neutralisiert ist, wobei gemäß einer Ausgestaltung des erfindungsgemäßen Verfahrens der Neutralisationsgrad der wasserabsorbierenden Polymergebilde in einem Bereich von 65 bis 80 Mol-%, besonders bevorzugt in einem Bereich von 70 bis 78 Mol-% und gemäß einer anderen Ausgestaltung in einem Bereich von 45 bis 65 Mol-%, besonders bevorzugt in einem Bereich von 50 bis 60 Mol-% liegen kann.

Weiterhin ist es gemäß einer bevorzugten Ausführungsform der erfindungsgemäßen Zusammensetzung bevorzugt, dass die wasserabsorbierenden Polymergebilde einen Innenbereich und einen den Innenbereich umgebenden Außenbereich aufweisen, wobei der Außenbereich einen höheren Vernetzungsgrad aufweist als der Innenbereich. Eine solcher inhomogener Vernetzungsgrad lässt sich dadurch erzielen, dass die wasserabsorbierenden Polymergebilde durch die Beschichtung mit einem geeigneten Oberflächennachvernetzer, welcher mindestens zwei funktionelle Gruppen aufweist, die mit den funktionellen Gruppen im Außenbereich des wasserabsorbierenden Polymergebildes reagieren können, nachvernetzt wird.

Einen Beitrag zur Lösung der eingangs genannten Aufgaben leistet auch ein Verfahren zur Herstellung einer wasserabsorbierenden Zusammensetzung, beinhaltend die Verfahrensschritte:
i) Bereitstellen eines wasserabsorbierenden Polymergebildes;
ii) Nachvernetzung des wasserabsorbierenden Polymergebildes,
iii) in Kontakt bringen des wasserabsorbierenden Polymergebildes
   a) mit mindestens einer Tanninfraktion, welche ein gemäß der hierin beschriebenen Testmethode bestimmtes Zahlenmittel des Molekulargewichtes von mindestens 1.000 g/mol besitzt, oder
   b) mit mindestens einer Tanninfraktion, welche ein gemäß der hierin beschriebenen Testmethode bestimmtes Gewichtsmittel des Molekulargewichtes von mindestens 1.000 g/mol besitzt, o
wobei der Verfahrensschritt iii) vor, während oder nach dem Verfahrensschritt ii) durchgeführt werden kann, vorzugsweise jedoch nach dem Verfahrensschritt ii) durchgeführt wird (was bedeutet, dass bereits oberflächennachvernetzte, wasserabsorbierende Polymergebilde mit den entsprechenden Tanninen in Kontakt gebracht werden). Bei erfindungsgemäßen Verfahren wird die mindestens eine Tanninfraktion in einer Menge in einem Bereich von 0,001 bis 10 Gew.-%, bezogen auf das Gesamtgewicht aus wasserabsorbierenden Polymergebilden, eingesetzt.

Im Verfahrensschritt i) des erfindungsgemäßen Verfahrens werden zunächst wasserabsorbierende Polymergebilde bereitgestellt.

Als wasserabsorbierende Polymergebilde werden dabei im Verfahrensschritt i) vorzugsweise Polymere bereitgestellt, welche erhalten wurden durch ein Verfahren umfassend die Verfahrensschritte:
a) radikalische Polymerisation säuregruppen-tragender, ethylenisch ungesättigter, gegebenenfalls teilneutralisierter Monomere in Gegenwart eines Vernetzers unter Bildung eines Hydrogels;
b) gegebenenfalls Zerkleinern des Hydrogels;
c) zumindest teilweises Trocknen des gegebenenfalls zerkleinerten Hydrogels unter Erhalt wasserabsorbierender Polymergebilde;
d) gegebenenfalls Zermahlen des so erhaltenen absorbierenden Polymergebildes und absieben auf eine gewünschte Partikelgrößenfraktion;
e) gegebenenfalls weitere Oberflächenmodifizierungen der so erhaltenen wasserabsorbierenden Polymergebilde.

Die im Verfahrensschritt a) erfolgende radikalische Polymerisation erfolgt vorzugsweise in wässriger Lösung, wobei diese wässrige Lösung neben Wasser als Lösungsmittel vorzugsweise
(α1) die ethylenisch ungesättigten, säuregruppen-tragenden Monomeren oder deren Salze, wobei Acrylsäure als säuregruppentragendes Monomer besonders bevorzugt ist,
(α2) gegebenenfalls monoethylenisch ungesättigte, mit (α1) copolymerisierbare Monomere,
(α3) den Vernetzer,
(α4) gegebenenfalls ein wasserlösliches Polymer, sowie
(α6) gegebenenfalls einen oder mehrere Hilfsmittel

### beinhaltet.

Als monoethylenisch ungesättigte, mit (α1) copolymerisierbare Monomere, wasserlösliche Polymere und Hilfsmittel sind wiederum diejenigen Verbindungen bevorzugt, die bereits eingangs im Zusammenhang mit den in der erfindungsgemäßen Zusammensetzung enthaltenen, wasserabsorbierenden Polymergebilden als mit (α1) copolymerisierbare Monomere, als wasserlösliche Polymere bzw., als Hilfsmittel genannt wurden.

Aus den vorgenannten Monomeren, Comonomeren, Vernetzern, wasserlöslichen Polymeren und Hilfsstoffen lassen sich die wasserabsorbierende Polymergebilde durch verschiedene Polymerisationsweisen herstellen. Beispielsweise sind in diesem Zusammenhang Massepolymerisation, die vorzugsweise in Knetreaktoren wie Extrudern erfolgt, Lösungspolymerisation, Spraypolymerisation, inverse Emulsionspolymerisation und inverse Suspensionspolymerisation zu nennen.

Bevorzugt wird die Lösungspolymerisation in Wasser als Lösungsmittel durchgeführt. Die Lösungspolymerisation kann kontinuierlich oder diskontinuierlich erfolgen. Aus dem Stand der Technik ist ein breites Spektrum von Variationsmöglichkeiten hinsichtlich Reaktionsverhältnisse wie Temperaturen, Art und Menge der Initiatoren als auch der Reaktionslösung zu entnehmen. Typische Verfahren sind in den folgenden Patentschriften beschrieben: US 4,286,082, DE 27 06 135, US 4,076,663, DE 35 03 458, DE 40 20 780, DE 42 44 548, DE 43 23 001, DE 43 33 056, DE 44 18 818.

Die Polymerisation wird wie allgemein üblich durch einen Initiator ausgelöst. Als Initiatoren zur Initiierung der Polymerisation können alle unter den Polymerisationsbedingungen Radikale bildende Initiatoren verwendet werden, die üblicherweise bei der Herstellung von Superabsorbern eingesetzt werden. Auch eine Initiierung der Polymerisation durch Einwirkung von Elektronenstrahlen auf die polymerisierbare, wässrige Mischung ist möglich. Die Polymerisation kann allerdings auch in Abwesenheit von Initiatoren der oben genannten Art durch Einwirkung energiereicher Strahlung in Gegenwart von Photoinitiatoren ausgelöst werden. Polymerisationsinitiatoren können in einer Lösung erfindungsgemäßer Monomere gelöst oder dispergiert enthalten sein. Als Initiatoren kommen sämtliche dem Fachmann bekannte in Radikale zerfallende Verbindungen in Betracht. Hierunter fallen insbesondere diejenigen Initiatoren, die bereits in der WO 2004/037903 A2 als mögliche Initiatoren genannt werden.

Besonders bevorzugt wird zur Herstellung der wasserabsorbierenden Polymergebilde ein Redoxsystem bestehend aus Wasserstoffperoxid, Natriumperoxodisulfat und Ascorbinsäure eingesetzt.

Auch die inverse Suspensions- und Emulsionspolymerisation kann zur Herstellung der Polymergebilde angewendet werden. Gemäß diesen Prozessen wird eine wässrige, teilneutralisierte Lösung der Monomeren (α1), und (α2), gegebenenfalls beinhaltend wasserlösliche Polymere und Hilfsstoffe, mit Hilfe von Schutzkolloiden und/oder Emulgatoren in einem hydrophoben, organischen Lösungsmittel dispergiert und durch Radikalinitiatoren die Polymerisation gestartet. Die Vernetzer sind entweder in der Monomerlösung gelöst und werden mit dieser zusammen dosiert oder aber separat und gegebenenfalls während der Polymerisation zugefügt. Gegebenenfalls erfolgt die Zugabe eines wasserlöslichen Polymeren (α4) als Pfropfgrundlage über die Monomerlösung oder durch direkte Vorlage in die Ölphase. Anschließend wird das Wasser azeotrop aus dem Gemisch entfernt und das Polymerisat abfiltriert.

Weiterhin kann sowohl bei der Lösungspolymerisation als auch bei der inversen Suspensions- und Emulsionspolymerisation die Vernetzung durch Einpolymerisation des in der Monomerlösung gelösten polyfunktionellen Vernetzers und/oder durch Reaktion geeigneter Vernetzer mit funktionellen Gruppen des Polymeren während der Polymerisationsschritte erfolgen. Die Verfahren sind beispielsweise in den Veröffentlichungen US 43 40 706, DE 37 13 601, DE 28 40 010 und WO 96/05234 A1 beschrieben.

Die bei der Lösungspolymerisation oder der inversen Suspensions- und Emulsionspolymerisation im Verfahrensschritt a) erhaltenen Hydrogele werden im Verfahrensschritt c) zumindest teilweise getrocknet.

Insbesondere im Falle der Lösungspolymerisation ist es jedoch bevorzugt, dass die Hydrogele vor der Trocknung zunächst in einem zusätzlichen Verfahrensschritt b) zerkleinert werden. Dieses Zerkleinern erfolgt durch dem Fachmann bekannte Zerkleinerungsvorrichtungen, wie etwa einem Fleischwolf.

Die Trocknung des Hydrogels erfolgt vorzugsweise in geeigneten Trocknern oder Öfen. Beispielhaft seien Drehrohröfen, Wirbelbetttrockner, Tellertrockner, Paddeltrockner oder Infrarottrockner genannt. Weiterhin ist es erfindungsgemäß bevorzugt, dass die Trocknung des Hydrogels im Verfahrensschritt c) bis zu einem Wassergehalt von 0,5 bis 50 Gew.-%, vorzugsweise von 1 bis 25 Gew.-%, besonders bevorzugt 2 bis 10 Gew.-% erfolgt, wobei die Trocknungstemperaturen üblicherweise in einem Bereich von 100 bis 200°C liegen.

Die im Verfahrensschritt c) erhaltenen, zumindest teilweise getrockneten wasserabsorbierenden Polymergebilde können, insbesondere dann, wenn sie durch Lösungspolymerisation erhalten wurden, in einem weiteren Verfahrensschritt d) noch zermahlen und auf die eingangs genannte Wunschkomgröße abgesiebt werden. Das Zermahlen der getrockneten, wasserabsorbierenden Polymergebilde erfolgt vorzugsweise in geeigneten, mechanischen Zerkleinerungsvorrichtungen, wie etwa einer Kugelmühle.

Im Anschluss an die Trocknung der Hydrogele und der gegebenenfalls erfolgten weiteren Konfektionierung der getrockneten wasserabsorbierenden Polymergebilde können diese in einem weiteren Verfahrenschritt e) im Oberflachenbereich modifiziert werden (ausgenommen von der im Verfahrensschritt e) durchgeführten Oberflächenmodifizierung ist die im nachfolgenden noch eingehender beschriebene Oberflächennachvernetzung gemäß Verfahrensschritt ii) und die Behandlung mit Tanninen im Verfahrensschritt iii)).

Als bevorzugte Modifizierungsmaßnahme sei hier das in Kontakt bringen des Außenbereiches der Polymergebilde mit einer Verbindung enthaltend Al³⁺-Ionen vor, während oder nach, vorzugsweise nach der Oberflächennachvernetzung gemäß Verfahrensschritt ii) zu nennen. Dabei ist es bevorzugt, dass die Verbindung enthaltend Al³⁺-Ionen in einer Menge in einem Bereich von 0,01 bis 30 Gew. -%, besonders bevorzugt in einer Menge in einem Bereich von 0,05 bis 20 Gew. -% und darüber hinaus bevorzugt in einer Menge in einem Bereich von 0,1 bis 5 Gew. -%, jeweils bezogen auf das Gewicht der wasserabsorbierenden Polymergebilde, mit den wasserabsorbierenden Polymergebilden in Kontakt gebracht wird.

Das in Kontakt bringen des Außenbereiches der wasserabsorbierenden Polymergebilde mit der Al³⁺-Ionen enthaltenden Verbindung erfolgt vorzugsweise dadurch, dass das Polymergebilde mit der Verbindung unter trockenen Bedingungen vermischt wird oder aber dadurch, dass die Polymergebilde mit einem Fluid F₁ umfassend ein Lösemittel, vorzugsweise Wasser, mit Wasser mischbare organische Lösemittel wie etwa Methanol oder Ethanol oder Mischungen aus mindestens zwei davon, sowie die Al³⁺-Ionen enthaltende Verbindung in Kontakt gebracht werden, wobei das in Kontakt bringen vorzugsweise durch Besprühen der Polymerteilchen mit dem Fluid F₁ und Vermischen erfolgt. In diesem Zusammenhang ist es weiterhin bevorzugt, dass das in Kontakt bringen der Polymergebilde mit dem Fluid F₁ enthaltend die Al³⁺-Ionen enthaltende Verbindung in einem zweistufigen Verfahren erfolgt. Dabei umfasst das zweistufige Verfahren einen ersten Mischvorgang, bei dem eine Vielzahl von Polymergebilden mit dem Fluid F₁ vermischt wird, und einem zweiten Mischvorgang, bei dem das Fluid F₁ im Inneren der Polymergebilde homogenisiert wird, wobei die Polymergebilde in dem ersten Mischvorgang mit einer Geschwindigkeit gemischt werden, dass die Bewegungsenergie der einzelnen Polymergebilde im Mittel größer ist als die Haftungsenergie zwischen den einzelnen Polymergebilden, und die Polymergebilde in dem zweiten Mischvorgang mit einer geringeren Geschwindigkeit als im ersten Mischvorgang durchmischt werden.

Durch die Behandlung der Polymergebilde mit dem Fluid F₁ beinhaltend die Al³⁺-Ionen enthaltende Verbindung durch das vorstehend beschriebene, zweistufige Verfahren können Polymergebilde mit verbesserten Absorptionseigenschaften erhalten werden.

Vorzugsweise ist dabei die Al³⁺-Ionen enthaltende Verbindung ohne Berücksichtigung von Kristallwasser in einer Menge in einem Bereich von 0,1 bis 50 Gew. - %, besonders bevorzugt in einer Menge in einem Bereich von 1 bis 30 Gew.-%, jeweils bezogen auf das Gesamtgewicht des Fluids F₁, in dem Fluid F₁ enthalten. Es ist weiterhin bevorzugt, dass das Fluid F₁ in einer Menge in einem Bereich von 0,01 bis 15 Gew.-%, besonders bevorzugt in einer Menge in einem Bereich von 0,05 bis 6 Gew.-%, jeweils bezogen auf das Gewicht der Polymergebilde, mit den Polymergebilden in Kontakt gebracht wird.

Bevorzugte Al³⁺-Ionen enthaltenden Verbindungen sind AlCl₃ x 6 H₂O, NaAl(SO₄)₂ x 12 H₂O, KAl(SO₄)₂ x 12 H₂O, Al₂(SO₄)₃ x 14-18 H₂O oder Aluminiumlactat.

Als weitere Oberflächenmodifizierungsmaßnahme sei an dieser Stelle das in Kontakt bringen der wasserabsorbierenden Polymergebilde mit anorganischen Partikeln, beispielsweise mit feinteiligem Siliziumdioxid, welches vorzugsweise in wässriger Suspension appliziert wird, oder mit Kieselsäuresol erwähnt.

Die vorstehend im Verfahrensschritt e) erfolgenden Modifizierungsmaßnahmen, insbesondere die Behandlung mit einer Al³⁺-Ionen enthaltenden Verbindung, können grundsätzlich auch während der Durchführung der Verfahrensschritte ii) und iii), nach Durchführung der Verfahrensschritte ii) und iii), nach Durchführung des Verfahrensschrittes ii) aber vor der Durchführung des Verfahrensschrittes iii) oder aber nach Durchführung des Verfahrensschrittes iii) aber vor der Durchführung des Verfahrensschrittes ii) erfolgen.

Im Verfahrensschritt ii) erfolgt die Oberflächennachvernetzung des wasserabsorbierenden Polymergebildes, bei der die wasserabsorbierenden Polymergebilde zunächst mit einem Oberflächennachvernetzer in Kontakt gebracht und dann erhitzt werden. Dabei wird der Nachvernetzer insbesondere dann, wenn er unter den Nachvernetzungsbedingungen nicht flüssig ist, vorzugsweise in Form eines Fluids F₂ umfassend den Nachvernetzer sowie ein Lösungsmittel mit den Polymerteilchen in Kontakt gebracht. Als Lösungsmittel werden dabei vorzugsweise Wasser, mit Wasser mischbare organische Lösungsmittel wie etwa Methanol, Ethanol, 1-Propanol, 2-Propanol oder 1-Butanol oder Mischungen aus mindestens zwei dieser Lösungsmittel eingesetzt, wobei Wasser als Lösungsmittel am meisten bevorzugt ist. Weiterhin ist es bevorzugt, dass der Nachvernetzer in dem Fluid F₂ in einer Menge in einem Bereich von 5 bis 75 Gew.-%, besonders bevorzugt 10 bis 50 Gew.-% und am meisten bevorzugt 15 bis 40 Gew.-%, bezogen auf das Gesamtgewicht des Fluids F₂, enthalten ist.

Das in Kontakt bringen des wasserabsorbierenden Polymergebildes mit dem Fluid F₂ beinhaltend den Nachvernetzer erfolgt im erfindungsgemäßen Verfahren vorzugsweise durch gutes Vermischen des Fluids F₂ mit dem wasserabsorbierenden Polymergebilde.

Geeignete Mischaggregate zum Aufbringen des Fluids F₂ sind zum Beispiel der Patterson-Kelley-Mischer, DRAIS-Turbulenzmischer, Lödigemischer, Ruberg-Mischer, Schneckenmischer, Tellermischer und Wirbelschichtmischer sowie kontinuierlich arbeitende senkrechte Mischer, in denen das Polymergebilde mittels rotierender Messer in schneller Frequenz gemischt wird (Schugi-Mischer). Auch können Mischvorrichtungen eingesetzt werden, in denen das Vermischen des Fluids F₂ mit dem wasserabsorbierenden Polymergebilde zumindest teilweise in einem sich drehenden Behälter erfolgt. Solche Mischvorrichtungen sind beispielweise von der Firma Lindor, Dordrecht, Niederlande erhältlich und werden beispielsweise unter den Produktbezeichnungen Lindor 70, Lindor 200, Lindor 500, Lindor 750, Lindor 1000, Lindor 1500, Lindor 2000, Lindor 2300, Lindor 4000, Lindor 7000, Lindor 8000, Lindor 12000, Lindor 14000 oder Lindor 25000 vertrieben.

Das wasserabsorbierende Polymergebilde wird in dem erfindungsgemäßen Verfahren bei der Nachvernetzung vorzugsweise mit höchstens 20 Gew. -%, besonders bevorzugt mit höchstens 15 Gew.-%, darüber hinaus bevorzugt mit höchstens 10 Gew. -%, darüber hinaus noch mehr bevorzugt mit höchstens 5 Gew. -% an Lösungsmittel, vorzugsweise Wasser, in Kontakt gebracht und am allermeisten bevorzugt mit weniger als 3 Gew.-%, jeweils bezogen auf das Gewicht des wasserabsorbierenden Polymergebildes.

Bei wasserabsorbierenden Polymergebilden in der Form von vorzugsweise kugelförmigen Teilchen ist es erfindungsgemäß weiterhin bevorzugt, dass das in Kontakt bringen derart erfolgt, dass lediglich der Außenbereich, nicht jedoch der innere Bereich der teilchenförmigen, wasserabsorbierenden Polymergebilde mit dem Fluid F₂ und somit dem Nachvernetzer in Kontakt gebracht werden.

Als Nachvernetzer, die im erfindungsgemäßen Verfahren eingesetzt werden, werden vorzugsweise Verbindungen verstanden, die mindestens zwei funktionelle Gruppen aufweisen, die mit funktionellen Gruppen eines Polymergebildes in einer Kondensationsreaktion (=Kondensationsvernetzer), in einer Additionsreaktion oder in einer Ringöffnungsreaktion reagieren können oder aber polyvalente Metallkationen, die mittels elektrostatischer Wechselwirkung zwischen dem polyvalenten Metallkation und den funktionellen Gruppen eines Polymergebildes eine Vernetzung des Polymergebildes ermöglichen. Als Nachvernetzer sind im erfindungsgemäßen Verfahren diejenigen bevorzugt, die in WO 2004/037903 A2 als Vernetzer der Vernetzerklassen II und IV genannt werden.

Unter diesen Verbindungen sind als Nachvernetzer besonders bevorzugt Kondensationsvernetzer wie beispielsweise Diethylenglykol, Triethylenglykol, Polyethylenglykol, Glyzerin, Polyglyzerin, Propylenglykol, Diethanolamin, Triethanolamin, Polyoxypropylen, Oxyethylen-Oxypropylen-Blockcopolymere, Sorbitanfettsäureester, Polyoxyethylensorbitanfettsäureester, Trimethylolpropan, Pentaerytrit, Polyvinylalkohol, Sorbitol, 1, 3-Dioxolan-2-on (Ethylencarbonat), 4-Methyl-1,3-dioxolan-2-on (Propylencarbonat), 4,5-Dimethyl-1,3-dioxolan-2-on, 4,4-Dimethyl-1,3-dioxolan-2-on, 4-Ethyl-1,3-dioxolan-2-on, 4-Hydroxymethyl-1,3-dioxolan-2-on, 1,3-Dioxan-2-on, 4-Methyl-1,3-dioxan-2-on, 4,6-Dimethyl-1,3-dioxan-2-on sowie 1,3-Dioxolan-2-on.

Nachdem die wasserabsorbierenden Polymergebilde mit dem Nachvernetzer bzw. mit dem Fluid beinhaltend den Nachvernetzer in Kontakt gebracht wurden, werden sie auf eine Temperatur im Bereich von 50 bis 300°C, vorzugsweise 75 bis 275°C und besonders bevorzugt 150 bis 250°C erhitzt, so dass, vorzugsweise wodurch, der Aussenbereich der Polymergebilde im Vergleich zum Innenbereich stärker vernetzt wird (=Nachvernetzung). Die Zeitdauer der Wärmebehandlung wird durch die Gefahr, dass das gewünschte Eigenschaftsprofil der wasserabsorbierenden Polymergebilde infolge von Hitzeeinwirkung zerstört wird, begrenzt.

Im Verfahrensschritt iii) des erfindungsgemäßen Verfahrens werden die wasserabsorbierenden Polymergebilde
a) mit mindestens einer Tanninfraktion, welche ein gemäß der hierin beschriebenen Testmethode bestimmtes Zahlenmittel des Molekulargewichtes von mindestens 1.000 g/mol, noch mehr bevorzugt von mindestens 1.100 g/mol und am meisten bevorzugt von mindestens 1.200 g/mol besitzt, oder
b) mit mindestens einer Tanninfraktion, welche ein gemäß der hierin beschriebenen Testmethode bestimmtes Gewichtsmittel des Molekulargewichtes von mindestens 1.000 g/mol, noch mehr bevorzugt von mindestens 1.100 g/mol und am meisten bevorzugt von mindestens 1.200 g/mol besitzt, oder
c) mit mindestens einer Tanninfraktion beinhaltend hydrolysierbare Gallotannine,
in Kontakt gebracht, wobei als Tannine insbesondere diejenigen Tannine bevorzugt sind, die bereits eingangs im Zusammenhang mit der erfindungsgemäßen Zusammensetzung als bevorzugte Tannine genannt wurden. Insbesondere sind demnach hydrolysierbare Tannine, insbesondere jedoch hydrolysierbare Gallotannine bevorzugt. Wie bereits vorstehend ausgeführt, kann dieses in Kontakt bringen mit den Tanninen vor, während oder nach der Durchführung des Verfahrensschrittes ii) erfolgen, wobei die Durchführung nach dem Verfahrensschritt ii) besonders bevorzugt ist. Gemäß einer besonders bevorzugten Ausführungsform des erfindungsgemäßen Verfahrens werden Tannine eingesetzt, welche sich von dem Grundgerüst der nachfolgenden Struktur I (β-1,2,3,4,6-Pentagalloyl-O-D-Glukose) 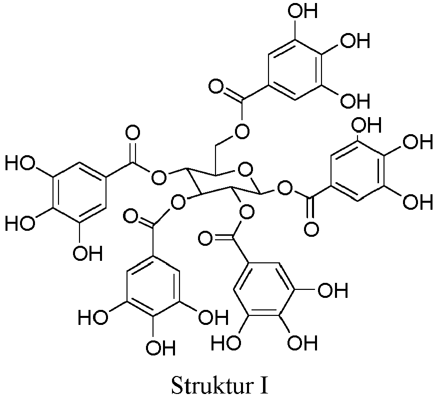 ableiten, wobei an mindestens eine der OH-Gruppen mindestens eines der an den Kohlenhydrat-Kern gebundenen Gallsäure-Moleküle (= Gallsäure-Molekül der ersten Ebene, gebunden als Galloylester), vorzugsweise an eine der OH-Gruppen in meta- oder in para-Position, vorzugsweise jedoch an mindestens eine der OH-Gruppen von 2, 3, 4 oder 5 der an den Kohlenhydrat-Kern gebundenen Gallsäure-Moleküle, mindestens ein weiteres Gallsäure-Molekül über eine Esterbindung (Digalloyl-Ester) gebunden ist (= Gallsäure-Molekül der zweiten Ebene). In diesem Zusammenhang ist es insbesondere bevorzugt, dass es sich bei dem Tannin um ein Tannin handelt, welches sich von dem Grundgerüst der Struktur I ableitet und welches mindestens ein, vorzugsweise zwei, drei, vier oder fünf Gallsäure-Moleküle der zweiten Ebene umfasst. Insbesondere jedoch ist es in diesem Zusammenhang bevorzugt, dass vorzugsweise mindestens 30 Gew.-%, besonders bevorzugt mindestens 50 Gew.-%, noch mehr bevorzugt mindestens 75 Gew.-% und am meisten bevorzugt mindestens 95 Gew.-% der in der wasserabsorbierenden Zusammensetzung enthaltenen Tannine ein Molekulargewicht von mehr als 940 g/mol, besonders bevorzugt mehr als 1.100 g/mol und darüber hinaus bevorzugt mehr als 1.200 g/mol aufweisen, wobei auch hier vorzugsweise ein Molekulargewicht von 10.000 g/mol, besonders bevorzugt von 5.000 g/mol nicht überschritten wird.

Erfindungsgemäß ist das Verfahren wenn die mindestens eine Tanninfraktion in einer Menge in einem Bereich von 0,001 bis 10 Gew.-% eingesetzt wird. Weiterhin bevorzugt ist es dabei, wenn die mindestens eine Tanninfraktion in einer Menge in einem Bereich von 0,1 bis 5 Gew.-% und am meisten bevorzugt in einer Menge in einem Bereich von 0,2 bis 2 Gew.-%, bezogen auf das Gesamtgewicht der wasserabsorbierenden Polymergebilde, eingesetzt wird. Besonders vorteilhaft im Zusammenhang mit der Farbstabilität der wasserabsorbierenden Zusammensetzung nach einem Kontakt mit Körperflüssigkeiten ist es jedoch, wenn die Tannine bzw. die Tanninfraktion in einer Menge von höchstens 0,75 Gew.-%, besonders bevorzugt höchstens 0,5 Gew.-% und am meisten bevorzugt höchstens 0,4 Gew.-%, jeweils bezogen auf das Gesamtgewicht der wasserabsorbierenden Polymergebilde, eingesetzt werden. Gemäß einer besonderen Ausführungsform der erfindungsgemäßen wasserabsorbierenden Zusammensetzung werden das Tannin bzw. die Tanninfraktion in einer Menge von 0,2 bis 0,4 Gew.-%, bezogen auf das Gesamtgewicht der wasserabsorbierenden Polymergebilde, eingesetzt.

Gemäß einer besonders bevorzugten Ausführungsform des erfindungsgemäßen Verfahrens wird die mindestens eine Tanninfraktion im Verfahrensschritt iii) in Form von Partikeln eingesetzt, insbesondere in Form eines Pulvers, wobei es in diesem Zusammenhang weiterhin bevorzugt ist, dass mindestens 50 Gew.-%, besonders bevorzugt mindestens 75 Gew.-%, noch mehr bevorzugt mindestens 95 Gew.-% und am meisten bevorzugt 100 Gew.-% der Partikel der mindestens einen Tanninfraktion eine Größe in einem Bereich von 10 nm bis 300 µm, besonders bevorzugt 50 nm bis 50 µm und am meisten bevorzugt 100 nm bis 10 µm besitzen.

Weiterhin ist es insbesondere im Zusammenhang mit dem Einsatz der mindestens einen Tanninfraktion in Form von Partikeln bevorzugt, dass die mindestens eine Tanninfraktion mindestens eine der folgenden Eigenschaften aufweist:
A) eine Dichte in einem Bereich von 0,2 bis 0,6 g/cm³, besonders bevorzugt in einem Bereich von 0,3 bis 0,45 g/cm³;
B) einen pH-Wert als 1 gew.-%iger Lösung in Wasser bei 20°C in einem Bereich von 2 bis 5, besonders bevorzugt in einem Bereich von 2,5-4;
C) einen Gehalt an freier Gallsäure von weniger als 0,5 Gew.-%, besonders bevorzugt weniger 0,1 Gew.-%, jeweils bezogen auf das Gesamtgewicht der Tanninfraktion;
D) eine Reinheit, angegeben als Tanninanteil, von mindestens 90 Gew.-%, besonders bevorzugt mindestens 95 Gew.-%, jeweils bezogen auf das Gesamtgewicht der Tanninfraktion.

Weiterhin bevorzugte Ausführungsformen der im erfindungsgemäßen Verfahren bevorzugt einsetzbaren Tanninfraktion weisen jede denkbare Kombination der vorstehenden Merkmale A bis D auf, wobei die Ausführungsformen der folgenden Merkmalskombinationen bevorzugt sind: A, B, C, D, AB, AC, AD, BC, BD, CD, ABC, ABD, ACD, BCD und ABCD.

Die im erfindungsgemäßen Verfahren einsetzbare Tanninfraktion kann aus geeigneten pflanzlichen Quelle isoliert oder von kommerziellen Anbietern bezogen werden, wie dies bereits eingangs im Zusammenhang mit der erfindungsgemäßen Zusammensetzung beschrieben worden ist.

Das in Kontakt bringen der gegebenenfalls bereits oberflächennachvernetzten, wasserabsorbierenden Polymergebilde mit der mindestens einen Tanninfraktion erfolgt vorzugsweise durch einfaches Vermischen der beiden Komponenten, wobei die mindestens eine Tanninfraktion vorzugsweise in partikulärer Form mit dem wasserabsorbierenden Polymergebilde vermischt wird. Grundsätzlich denkbar ist jedoch auch, die Tanninfraktion in Form einer Lösung oder einer Suspension, beispielsweise in Form einer wässrigen Lösung, mit dem wasserabsorbierenden Polymergebilde zu vermischen. Wie bereist vorstehend ausgeführt, hat sich jedoch insbesondere das Vermischen der mindestens einen Tanninfraktion in partikulärer Form, insbesondere in Form eines Pulvers, mit den wasserabsorbierenden Polymergebilden als vorteilhaft erwiesen.

Geeignete Mischaggregate zum Vermischen der mindestens einen Tanninfraktion und der wasserabsorbierenden Polymergebilde sind beispielsweise der Patterson-Kelley-Mischer, DRAIS-Turbulenzmischer, Lödigemischer, Ruberg-Mischer, Schneckenmischer, Tellermischer und Wirbelschichtmischer sowie kontinuierlich arbeitende senkrechte Mischer, in denen das Polymergebilde mittels rotierender Messer in schneller Frequenz gemischt wird (Schugi-Mischer). Denkbar ist auch hier der bereits im Zusammenhang mit der Oberflächennachvernetzung beschriebene Einsatz von Mischvorrichtungen, in denen das Vermischen der pulverförmigen Tannine oder der Tanninlösungen mit den wasserabsorbierenden Polymergebilden zumindest teilweise in einem sich drehenden Behälter erfolgt.

Gemäß einer bevorzugten Ausführungsform des erfindungsgemäßen Verfahrens beinhaltet dieses den weiteren Verfahrensschritt des
iv) in Kontakt bringens der wasserabsorbierenden Polymergebilde mit einem oder mehreren Komplexbildner(n),
wobei der Verfahrensschritt iv) ebenso wie der Verfahrensschritt iii) vor, während oder nach dem Verfahrensschritt ii) durchgeführt werden kann, vorzugsweise jedoch nach dem Verfahrensschritt ii) durchgeführt wird (was bedeutet, dass bereits oberflächennachvernetzte, wasserabsorbierende Polymergebilde mit den entsprechenden Komplexbildnern in Kontakt gebracht werden). Hinsichtlich des Einsatzes der Tanninfraktion ist es grundsätzlich möglich
- zunächst die Tanninfraktion und dann den Komplexbildner mit den wasserabsorbierenden Polymergebilden in Kontakt zu bringen,
- zunächst den Komplexbildner und dann die Tanninfraktion mit den wasserabsorbierenden Polymergebilden in Kontakt zu bringen, oder
- den Komplexbildner zusammen mit der Tanninfraktion mit den wasserabsorbierenden Polymergebilde in Kontakt zu bringen.

Als Komplexbildner sind diejenigen Komplexbildner bevorzugt, die bereits eingangs im Zusammenhang mit der erfindungsgemäßen wasserabsorbierenden Zusammensetzung als bevorzugte Komplexbildner genannt wurden. Weiterhin ist es erfindungsgemäß bevorzugt, dass der Komplexbildner in einer Menge in einem Bereich von 0,001 bis 5 Gew.-%, besonders bevorzugt in einem Bereich von 0,01 bis 1 Gew.-% und am meisten bevorzugt in einer Menge in einem Bereich von 0,05 bis 0,5 Gew.-%, jeweils bezogen auf das Gesamtgewicht der wasserabsorbierenden Polymergebilde, eingesetzt wird.

Das in Kontakt bringen der wasserabsorbierenden Polymergebilde mit der Tanninfraktion und dem Komplexbildner kann auf verschiedene Weise erfolgen. Gemäß einer bevorzugten Ausführungsform des erfindungsgemäßen Verfahrens wird die Tanninfraktion trocken in partikulärer Form mit dem wasserabsorbierenden Polymergebilde vermischt und der Komplexbildner wird in Form einer wässrigen Lösung eingesetzt. Auf diese Weise wird das Stauben der wasserabsorbierenden Zusammensetzung nach dem Zusatz des trocken zugemischten Tannins deutlich reduziert. Denkbar ist jedoch auch, eine wässrige Lösung beinhaltend die Tanninfraktion und den Komplexbildner mit dem wasserabsorbierenden Polymergebilde in Kontakt zu bringen. In diesem Zusammenhang ist es insbesondere bevorzugt, dass eine solche wässrige Lösung die Tanninfraktion in einer Konzentration in einem Bereich von 5 bis 50 Gew.-%, besonders bevorzugt 7,5 bis 30 Gew.-% und am meisten bevorzugt 10 bis 20 Gew.-%, jeweils bezogen auf das Gesamtgewicht der wässrigen Lösung enthält, wobei es insbesondere vorteilhaft ist, die Konzentration kleiner oder gleich 20 Gew.-% zu wählen, da dies zu einer besonders gleichmäßigen Verteilung der Tanninfraktion auf der Oberfläche der wasserabsorbierenden Polymergebilde führt. Die Konzentration an Komplexbildner in der wässrigen Lösung liegt vorzugsweise in einem Bereich von 20 bis 60 Gew.-%, besonders bevorzugt in einem Bereich von 30 bis 50 Gew.-%, jeweils bezogen auf das Gesamtgewicht der wässrigen Lösung. Neben der Zugabe einer wässrigen Lösung, welche die Tanninfraktion und den Komplexbildner gemeinsam beinhaltet, ist es auch möglich, getrennte Lösungen (also eine wässrige Lösung beinhaltend den Komplexbildner und eine wässrige Lösung beinhaltend die Tanninfraktion) zuzusetzen, wobei es in diesem Zusammenhang bevorzugt ist, dass die Konzentration an Tanninfraktion und Komplexbildner in den jeweiligen, wässrigen Lösungen in den vorstehend für die gemeinsame Lösung angegebenen Konzentrationsbereichen liegt. Der Begriff "wässrige Lösung", wie er hierin verwendet wird, umfasst jegliche Lösungen, bei denen das Lösungsmittel bzw. das Lösungsmittelgemisch zu mindestens 50 Gew.-%, besonders bevorzugt mindestens 75 Gew.-% und am meisten bevorzugt mindestens 90 Gew.-%, jeweils bezogen auf das Gesamtgewicht des Lösungsmittels, auf Wasser basiert.

Das Aufbringen der getrennt zugegebenen Lösungen, der gemeinsamen Lösung, oder der trocken zugemischten Tanninfraktion und der wässrigen Lösung des Komplexbildners kann wiederum durch geeignete Mischaggregate, wie etwa dem Patterson-Kelley-Mischer, DRAIS-Turbulenzmischer, Lödigemischer, Ruberg-Mischer, Schneckenmischer, Tellermischer und dem Wirbelschichtmischer sowie durch kontinuierlich arbeitende senkrechte Mischer, in denen das Polymergebilde mittels rotierender Messer in schneller Frequenz gemischt wird (Schugi-Mischer), erfolgen. Auch können zu diesem Zwecke Mischvorrichtungen eingesetzt werden, in denen das Vermischen des wasserabsorbierenden Polymergebildes mit den trocken zugemischten Komponenten oder den wässrigen Lösungen zumindest teilweise in einem sich drehenden Behälter erfolgt.

Im Zusammenhang mit dem Einsatz von Komplexbildnern entspricht es einer besonderen Ausführungsform eines erfindungsgemäßen Verfahrens zur Herstellung einer wasserabsorbierenden Zusammensetzung, dass dieses die Verfahrensschritte:
i) Bereitstellen eines wasserabsorbierenden Polymergebildes;
ii) Nachvernetzung des wasserabsorbierenden Polymergebildes;
iii) in Kontakt bringen des wasserabsorbierenden Polymergebildes mit mindestens einer Tanninfraktion, beinhaltend ein Tannin, wobei es sich bei dieser Tanninfraktion vorzugsweise um die vorstehend genannten Tanninfraktionen (Tanninfraktion mit einem gemäß der hierin beschriebenen Testmethode bestimmten Gewichtsmittel des Molekulargewichtes von mindestens 1.000 g/mol, Tanninfraktion mit einem gemäß der hierin beschriebenen Testmethode bestimmten Zahlenmittel des Molekulargewichtes von mindestens 1.000 g/mol oder Tanninfraktion beinhaltend hydrolysierbare Gallotannine) handelt und wobei in diesem Falle die Tanninfraktionen in aufgereinigter Form oder aber in Form von Pflanzenextrakten, insbesondere Tee-Extrakten, eingesetzt werden können;
iv) in Kontakt bringen der wasserabsorbierenden Polymergebilde mit dem einem oder den mehreren Komplexbildnern;
beinhaltet, wobei die Verfahrensschritte iii) und iv) vor, während oder nach dem Verfahrensschritt ii) durchgeführt werden können, vorzugsweise jedoch nach dem Verfahrensschritt ii) durchgeführt werden (was bedeutet, dass bereits oberflächennachvernetzte, wasserabsorbierende Polymergebilde mit dem entsprechenden Tannin und dem Komplexbildner in Kontakt gebracht werden).

Nachdem die wasserabsorbierenden Polymergebilde mit der mindestens einen Tanninfraktion und gegebenenfalls dem mindestens einen Komplexbildner in Kontakt gebracht worden sind, kann die so erhaltene Zusammensetzung noch weiteren Modifizierungsmaßnahmen unterworfen werden. Denkbar ist hier insbesondere der Zusatz der eingangs im Zusammenhang mit der erfindungsgemäßen Zusammensetzung genannten Binder.

Einen Beitrag zur Lösung der eingangs genannten Aufgaben leistet auch eine wasserabsorbierende Zusammensetzung, welche durch das vorstehend beschriebene, erfindungsgemäße Verfahren erhältlich ist.

Es ist besonders bevorzugt, dass die eingangs beschriebene, erfindungsgemäße Zusammensetzug und auch die durch das vorstehend beschriebene Verfahren erhältliche, erfindungsgemäße Zusammensetzung mindestens eine der folgenden Eigenschaften aufweist:
(β1) eine nach ERT 441.2-02 bestimmte Retention von mindestens 20g/g, vorzugsweise mindestens 25g/g und besonders bevorzugt in einem Bereich von 25 bis 50 g/g;
(β2) eine (im Falle von Partikeln für die gesamte Partikelgrößenfraktion) bestimmte Absorption gegen einen Druck von 4830 Pa (0,7 psi) (50 g/cm²) nach ERT 442.2-02 von mindestens 15 g/g, besonders bevorzugt von mindestens 20 g/g;
(β3) einen Neutralisationsgrad von höchstens 78 Mol-%, besonders bevorzugt von höchstens 70 Mol-% und mindestens 50 Mol-%.

Offenbart, aber nicht beansprucht wird ein Verbund beinhaltend eine erfindungsgemäße wasserabsorbierende Zusammensetzung oder eine durch das erfindungsgemäße Verfahren erhältliche, wasserabsorbierende Zusammensetzung und ein Substrat.

Es ist dabei bevorzugt, dass die erfindungsgemäße, wasserabsorbierende Zusammensetzung und das Substrat miteinander fest verbunden sind. Als Substrate sind Folien aus Polymeren, wie beispielsweise aus Polyethylen, Polypropylen oder Polyamid, Metalle, Vliese, Fluff, Tissues, Gewebe, natürliche oder synthetische Fasern, oder andere Schäume bevorzugt. Weiterhin ist es erfindungsgemäß bevorzugt, dass der Verbund mindestens einen Bereich beinhaltet, welcher die erfindungsgemäße, wasserabsorbierende Zusammensetzung in einer Menge in einem Bereich von etwa 15 bis 100 Gew.-%, vorzugsweise etwa 30 bis 100 Gew.-%, besonders bevorzugt von etwa 50 bis 99,99 Gew.-%, ferner bevorzugt von etwa 60 bis 99,99 Gew.-% und darüber hinaus bevorzugt von etwa 70 bis 99 Gew.-%, jeweils bezogen auf das Gesamtgewicht des betreffenden Bereichs des Verbundes, beinhaltet, wobei dieser Bereich vorzugsweise eine Größe von mindestens 0,01 cm³, vorzugsweise mindestens 0,1 cm³ und am meisten bevorzugt mindestens 0,5 cm³ aufweist.

In einer Ausgestaltung des offenbarten, aber nicht beanspruchten Verbundes handelt es sich um einen flächenförmigen Verbund, wie er in der WO 02/056812 A1 als "absorbent material" beschrieben ist..

Ebenso offenbart aber nicht beansprucht wird hier ein Verfahren zur Herstellung eines Verbundes, wobei die erfindungsgemäße, wasserabsorbierende Zusammensetzung bzw. die durch das erfindungsgemäße Verfahren erhältliche, wasserabsorbierende Zusammensetzung und ein Substrat und gegebenenfalls ein Zusatzstoff miteinander in Kontakt gebracht werden. Als Substrate werden vorzugsweise diejenigen Substrate eingesetzt, die bereits vorstehend im Zusammenhang mit dem offenbartem Verbund genannt wurden.

Nach einem anderen Aspekt der nicht beanspruchten Offenbarung ist der Verbund als ein Hygieneartikelcore ausgebildet, das, jeweils bezogen auf das Hygieneartikelcore mindestens 30 Gew.- %, vorzugsweise mindestens 50 Gew.-% und darüber hinaus bevorzugt mindestens 70 Gew.- % der erfindungsgemäßen wasserabsorbierenden Zusammensetzung und mindestens 1 Gew.-%, vorzugsweise mindestens 5 Gew.-% und darüber hinaus bevorzugt mindestens 10 Gew.- % des Substrats und gegebenenfalls noch weitere übliche Hilfs- und/oder Haftmittel beinhaltet, wobei die Summe der Gewichtsprozente der einzelnen im Hygieneartikelcore beinhalteten Komponenten an 100 Gew.-% ergibt. Als Substrat im Zusammenhang mit dem Hygieneartikelcore sind besonders Materialien bevorzugt, die zur Fixierung der meist als Teilchen vorliegenden erfindungsgemäßen superabsorbierenden Zusammensetzung dienen. Hierbei kann es sich um Fasern oder Gewirke oder Gewebe sowie Netze handeln. Es ist weiterhin möglich, dass die beispielsweise als Pulver und damit teilchenförmig vorliegende wasserabsorbierende Zusammensetzung durch ein Haftmittel wie einen Klebstoff mit dem Substrat verbunden ist. Gleichfalls entspricht es einer Ausgestaltung, dass das Substrat so ausgestaltet ist, dass die wasserabsorbierende Zusammensetzung in Ausnehmung des Substrats aufgenommen wird. Übliche, ebenfalls in dem Hygieneartikelcore einarbeitbare Hilfsmittel sind beispielsweise kosmetische Stoffe, Stoffe, die die Hautverträglichkeit erhöhen, Desinfektionsmittel, antimikrobielle Substanzen und dergleichen.

In einem weiteren Aspekt betrifft die nicht beanspruchte Offenbarung Hygieneartikel, beinhaltend eine flüssigkeitsdurchlässige Oberschicht, eine flüssigkeitsundurchlässige Unterschicht und einen zwischen der Oberschicht und der Unterschicht angeordneten erfindungsgemäßen Verbund. Als Hygieneartikel kommen sowohl Damenhygieneartikel, Erwachsenen-Inkontinenzprodukte als auch Windeln für Säuglinge, Babys und Kleinkinder in Betracht. Bevorzugt ist es, dass der Hygieneartikel ein vorstehend beschriebenes Hygieneartikelcore beinhaltet. Als flüssigkeitsdurchlässige Oberschicht kommen grundsätzlich alle dem Fachmann hierfür bekannten und geeignet erscheinenden Gewebe, Gelege und Gewirklagen, die meist aus Zellstoffen oder Zellstoffderivaten bestehen, die gegebenenfalls mit Kunststoffen wie Polypropylen oder Polyethylen bondiert sind, in Betracht. Auch als flüssigkeitsundurchlässige Unterschicht werden die in der Industrie dem Fachmann geläufigen meist ebenfalls aus einem Zellstoff oder Zellstoffderivat, - gelege, -gewirk, oder -gestrick bestehenden Vliese eingesetzt, wobei diese im Allgemeinen mit einer Kunststoffschicht, meist aus Polypropylen oder Polyethylen, abgelichtet sind.

Auch die Verwendung der erfindungsgemäßen, wasserabsorbierenden Zusammensetzung in Schäumen, Formkörpern, Fasern, Folien, Filmen, Kabeln, Dichtungsmaterialien, flüssigkeitsaufnehmende Hygieneartikel insbesondere in Hygieneartikeln wie Windeln oder Damenbinden, sowie die Verwendung der Superabsorberpartikel als Träger für pflanzen- oder pilzwachstumsregulierenden Mittel oder Pflanzenschutzwirkstoffe liefern einen Beitrag zur Lösung der eingangs genannten Aufgaben. Bei der Verwendung als Träger für pflanzen- oder pilzwachstumsregulierende Mittel oder Pflanzenschutzwirkstoffe ist es bevorzugt, dass die pflanzen- oder pilzwachstumsregulierende Mittel oder Pflanzenschutzwirkstoffe über einen durch den Träger kontrollierten Zeitraum abgegeben werden können.

Die Erfindung wird nachfolgend anhand von Testmethoden und Figuren und Beispielen näher erläutert.

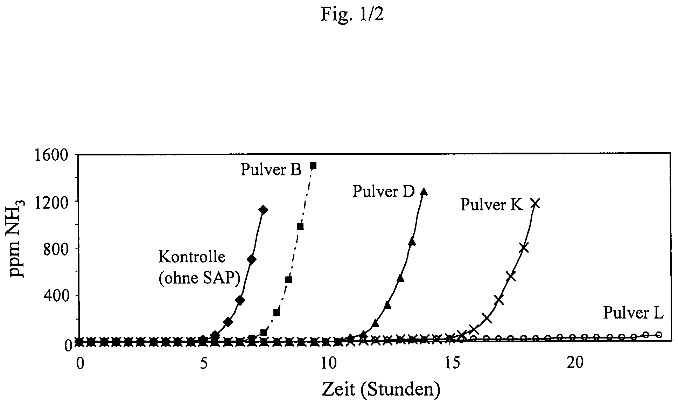

Es zeigt die Figur 1 den geruchsbindenden Effekt des Einsatzes von Tanninen bei Superabsorbern mit unterschiedlichem Neutralisationsgrad.

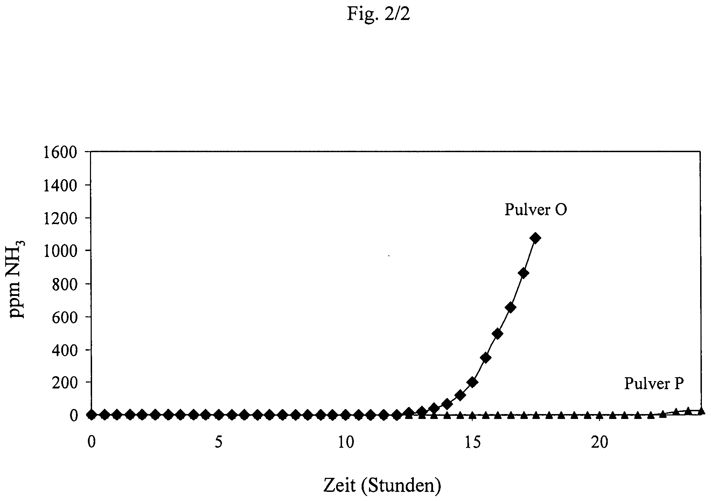

Es zeigt die Figur 2 den geruchsbindenden Effekt des kombinierten Einsatzes von Tanninen und Komplexbildnern.

### Testmethoden

### Allgemein

Sofern nicht nachfolgend andere Testmethoden vorgegeben werden, wird auf die dem Fachmann allgemein bekannten und üblich erscheinenden Testmethoden zurückgegriffen, wobei insbesondere Testmethoden der EDANA (European Diaper and Nonwoven Association) Anwendung finden, die allgemein als "ERT-Methoden" angegeben werden.

### Bestimmung des Molekulargewichtes und der Polydispersität der Tannine

Die Bestimmung des Gewichtsmittels und auch des Zahlenmittels des Molekulargewichtes sowie der Polydispersität der Tannine, insbesondere der erfindungsgemäß bevorzugt einzusetzenden hydrolysierbaren Tannine erfolgt vorzugsweise über eine MALDI-TOF-MS-Analyse, wie sie beispielsweise von C. Mane et al. , "Assessment of the Molecular Weight Distribution of Tannin Fractions through MALDI-TOF MS Analysis of Protein-Tannin Complexes", Analytical Chemistry, Vol. 79 (6), Seiten 2.239 bis 2.248 (2007) beschrieben ist.

### Bestimmung des Molekurlargewichtes von Gallotanninen

Das Molekulargewicht von Gallotanninen kann neben der vorstehend beschriebenen Testmethode auch durch sogenannte *"straight phase*"-HPLC-Verfahren bestimmt werden. Bei diesem Verfahren werden zwei verschiedene, mobile Phasen (Mobile Phase A: n-Hexan; mobile Phase B: 750 ml Methanol, 250 ml THF und 2,5 g Zitronensäure) zeitlich in unterschiedlichen relativen Mengen miteinander kombiniert. Die zeitliche Abfolge der Kombination der mobilen Phasen A und B wurde wie folgt gewählt:

| Zeit [min] | Mobile Phase A [%] | Mobile Phase B [%] |
|---|---|---|
| 0,00 | 80 | 20 |
| 15,00 | 50 | 50 |
| 30,00 | 35 | 65 |
| 31,00 | 20 | 80 |
| 35,00 | 0 | 100 |
| 40,00 | 0 | 100 |
| 41,00 | 80 | 20 |
| 45,01 | 80 | 20 |

Folgende Geräte und Materialien werden eingesetzt:
- HPLC-Pumpsystem 325 von KONTRON-Instruments
- DEGASYS DG-1300 UniFlows
- HPLC-Autosampler 465 BIO-TEK Systems
- HPLC-Detector 430 von KONTRON-Instruments
- KromaSystem 2000 Software
- LichroCart 250-4, LichroSorb Si60 Merck 1.51351.0001#
- Säule: LiChrosorb SI 60 Merck 1.51351.0001 LiChroCART 250-4 OB281194

Zur Durchführung der Chromatographie werden 100 mg der zu analysierenden Gallotannin-Probe in einer 100 ml Messflasche in Methanol gelöst. Die Probe wird in die HPLC-Vorrichtung injiziert. Folgende Parameter werden eingehalten:
Flussrate: 2 ml/min
Golflente (Wellenlänge): 280 nm
Bereich: 0,500
Säulenofen: 35°C

Als Standart wird reine, kommerziell erhältliche β-1,2,3,4,6-Pentagalloyl-O-D-Glukose eingesetzt.

### Bestimmung der L,a,b-Farbwerte

Die Bestimmung der L,a,b-Farbwerte erfolgt mittels eines Spektralkolorimeters *"Hunter LabScan XE"* (Hunter Associates Laboratory, Reston, VA, USA) bei folgenden Einstellungen:

| | |
|---|---|
| "Mode" | 0/45 |
| "Area View" | 44,5 mm |
| "Port Size" | 50,8 mm |
| "UV-Filter" | Nominal |

Vor jeder Messung wird der LabScan XE kalibriert, in dem zunächst die zum Gerätezubehör gehörende schwarze Glasplatte zwischen Probenteller und Messöffnung eingeklemmt wird, wobei die Glasplatte auf eine Petrischale (Durchmesser 100 mm, Tiefe: 15 mm) gelegt und durch Betätigung des "OK"-Schalters die Kalibrierung mit der schwarzen Glasplatte vollzogen wird. Anschließend wird auf die gleiche Art und Weise die weiße Standardplatte auf die Petrischale gelegt und erneut durch Betätigung des "OK"-Schalters die Kalibierung vollzogen.

Nachdem die Kalibierung durchgeführt wurde, wird der "Read Std"-Schalter gedrückt, um die Funktionsfähigkeit des Messgerätes zu prüfen, wobei dabei die Standardplatte noch nicht entfernt wird. Anschließend wird zur Messung der L,a,b-Werte für die Standardplatte der "Read"-Schalter betätigt. Anschließend wird die Standardplatte entfernt.

Für die Messung wurden 3 g der zu analysierenden, wasserabsorbierenden Zusammensetzung auf dem Boden einer Petrischale fein verteilt. 90 g einer FeCl₂-Lösung mit der Konzentration 0,0033% FeCl₂ in 0,9%iger NaCl-Lösung wurden schrittweise hinzugegeben und homogenisiert. Nach gleichmäßiger Quellung und Färbung wurde das überquollene Gel abgestrichen und die Probe unverzüglich im Hunter Colour Gerät gemessen, um das Aufreißen des Gels nach Abtrocknung zu vermeiden. Zur besseren Differenzierung wurde in dieser Messung unter jede Petrischale ein weißes Blatt gehalten. Mit diesem weißen Blatt wurden als Kontrolle folgende Werte ermittelt: L = 96,97; a = 4,02; b = -21,68.

### Beispiele

### Beispiel 1a Bereitstellung eines wasserabsorbierenden Polymergebildes (Verfahrensschritt i))

Eine Monomerenlösung bestehend aus 300 g Acrylsäure, die zu 70 Mol-% mit Natronlauge neutralisiert wurde, 441 g Wasser, 0,9 g Polyethylenglykol-300-diacrylat und 1,35 g Allyloxypolyethylenglykolacrylsäureester wird durch Spülen mit Stickstoff vom gelösten Sauerstoff befreit und auf die Starttemperatur von 4°C abgekühlt. Nach Erreichen der Starttemperatur wurde die Initiatorlösung (0,3 g Natriumperoxydisulfat in 10 g H₂O, 0,07 g 35%ge Wasserstoffperoxidlösung in 10 g H₂O und 0,015 g Ascorbinsäure in 2 g H₂O) zugesetzt. Nachdem die Endtemperatur von ca. 100°C erreicht war, wurde das entstandene Gel gewölft, so dass ein Granulat mit etwa 1 bis 3mm großen Teilchen erhalten wurde. Der Wassergehalt betrug ca. 50 %. Diese Teilchen wurden bei 150°C 120 Minuten lang getrocknet. Das getrocknete Polymerisat wurde grob zerstoßen, gemahlen und auf ein Pulver mit einer Partikelgröße von 150 bis 850 µm gesiebt und ein wasserabsorbierendes Polymergebilde (Pulver A) mit einer mittleren Teilchengröße nach ERT 420.2-02 von 520 µm erhalten. Der nach ERT 430.1-99 bestimmte Wassergehalt betrug 5%.

### Beispiel 2a Oberflächennachvernetzung eines wasserabsorbierenden Polymergebildes (Verfahrensschritt ii))

Zur Nachvernetzung wurden 100g des oben erhaltenen Pulvers A unter kräftigen Rühren mit einer Lösung aus 1 g 1,3-Dioxolan-2-on, 3g Wasser vermischt und anschließend für 30 Minuten in einem Ofen, der auf 180°C temperiert war, erhitzt. Das so erhaltene, oberflächennachvernetzte, wasserabsorbierende Polymergebilde (Pulver B) hatte eine mittlere Teilchengröße nach ERT 420.2-02 von 525 µm. Der im Pulver B nach ERT 430.1-99 bestimmte Wassergehalt betrug 4,5%.

### Beispiel 1b Bereitstellung eines wasserabsorbierenden Polymergebildes (Verfahrensschritt i))

Eine Monomerenlösung bestehend aus 300 g Acrylsäure, die zu 55 Mol-% mit Natronlauge neutralisiert wurde, 491 g Wasser, 0,9 g Polyethylenglykol-300-diacrylat und 1,35 g Allyloxypolyethylenglykolacrylsäureester wird durch Spülen mit Stickstoff vom gelösten Sauerstoff befreit und auf die Starttemperatur von 4°C abgekühlt. Nach Erreichen der Starttemperatur wurde die Initiatorlösung (0,3 g Natriumperoxydisulfat in 10 g H₂O, 0,07 g 35%ge Wasserstoffperoxidlösung in 10 g H₂O und 0,015 g Ascorbinsäure in 2 g H₂O) zugesetzt. Nachdem die Endtemperatur von ca. 100°C erreicht war, wurde das entstandene Gel gewölft, so dass ein Granulat mit etwa 1 bis 3mm großen Teilchen erhalten wurde. Der Wassergehalt betrug ca. 50 %. Diese Teilchen wurden bei 150°C 120 Minuten lang getrocknet. Das getrocknete Polymerisat wurde grob zerstoßen, gemahlen und auf ein Pulver mit einer Partikelgröße von 150 bis 850 µm gesiebt und ein wasserabsorbierendes Polymergebilde (Pulver C) mit einer mittleren Teilchengröße nach ERT 420.2-02 von 520 µm erhalten. Der nach ERT 430.1-99 bestimmte Wassergehalt betrug 5%.

### Beispiel 2b Oberflächennachvernetzung eines wasserabsorbierenden Polymergebildes (Verfahrensschritt ii))

Zur Nachvernetzung wurden 100g des oben erhaltenen Pulvers C unter kräftigen Rühren mit einer Lösung aus 1 g 1,3-Dioxolan-2-on, 3g Wasser vermischt und anschließend für 30 Minuten in einem Ofen, der auf 180°C temperiert war, erhitzt. Das so erhaltene, oberflächennachvernetzte, wasserabsorbierende Polymergebilde (Pulver D) hatte eine mittlere Teilchengröße nach ERT 420.2-02 von 525 µm. Der im Pulver D nach ERT 430.1-99 bestimmte Wassergehalt betrug 4,5%.

### Beispiel 3 In Kontakt bringen eines wasserabsorbierenden Polymergebildes mit Tanninen (Verfahrensschritt iii))

Das im Beispiel 2a erhaltene, oberflächennachvernetzte, wasserabsorbierende Polymergebilde (Pulver B) wurde mit verschiedenen partikulären, hydrolysierbaren Gallotanninen vermischt (siehe die Angaben in den nachfolgenden Tabellen 1 und 2). Dabei wurden die erfindungsgemäßen Zusammensetzungen gemäß den Pulvern E bis G und H bis J erhalten:

**Tabelle 1**

| | Pulver B (Vergleich) | Pulver E | Pulver F | Pulver G |
|---|---|---|---|---|
| Tannin | Floctan^{®} 1 | Floctan^{®} 1 | Floctan^{®} 1 | Floctan^{®} 1 |
| Menge [Gew.-%] | 0 | 0,5 | 0,75 | 1,0 |
| TB [g/g] | 30,0 | 29,0 | 29,0 | 29,0 |
| AAP₄₈₃₀ Pa (0,7 psi) [g/g] | 21,1 | 18,6 | 18,9 | 18,4 |

**Tabelle 2**

| | Pulver B (Vergleich) | Pulver H | Pulver I | Pulver J |
|---|---|---|---|---|
| Tannin | Brewtan^{®} | Brewtan^{®} | Brewtan^{®} | Brewtan^{®} |
| Menge [Gew.-%] | 0 | 0,5 | 0,75 | 1,0 |
| TB [g/g] | 30,0 | 29,7 | 28,0 | 28,1 |
| AAP₄₈₃₀ Pa (0,7 psi) [g/g] | 21,1 | 20,0 | 19,5 | 18,5 |

Den Ergebnissen der Tabellen 1 und 2 ist zu entnehmen, dass der erfindungsgemäße Zusatz der hydrolysierbaren Tannine das Absorptionsverhalten der wasserabsorbierenden Polymergebilde kaum nennenswert beeinflusst.

### Beispiel 4 Bestimmung der geruchsbindenden Eigenschaften der erfindungsgemäßen Zusammensetzungen

In einem weiteren Versuch wurden die Pulver B und D erneut mit 0,5 Gew.-% Floctan^{®} 1 vermischt, wobei zusätzlich 1.000 ppm Polyethylenglykol (Molekulargewicht = 10.000 g/mol), appliziert in Form einer 4,6 gew.-%igen Lösung, zugesetzt wurde. Dabei wurden die Pulver K (ausgehend von Pulver B, 70 % neutralisiert) und L (ausgehend von Pulver D, 55 % neutralisiert) erhalten.

Von den Pulvern B, D, K und L wurden die geruchsbindenden Eigenschaften bestimmt.

Die Bestimmung der geruchsbindenden Eigenschaften der erfindungsgemäßen Zusammensetzungen erfolgte über die Bestimmung der Ammoniakfreisetzung der wasserabsorbierenden Zusammensetzung.

Dazu wurden *Proteus mirabilis*-Zellen über Nacht bei 37°C auf Caso-Schrägagar angezogen. Pro Röhrchen wurde mit 5 ml künstlichem Urin abgeschwemmt. Die Bakteriensuspension wurde mit künstlichem Urin und 1 g/l Fleischextrakt sowie 1 g/l Pepton auf einen Keimgehalt von ungefähr 10⁵ KBE/ml eingestellt (KBE = Koloniebildende Einheiten).

Jeweils 1 g der wasserabsorbierenden Zusammensetzung wurden mit 33 ml des mit Bakterien versetzten, künstlichen Urins enthaltend 1 g/l Fleischextrakt und 1 g/l Pepton versetzt. Als Kontrolle wurde zum einen ein Ansatz ohne eine erfindungsgemäße Zusammensetzung sowie eine wasserabsorbierende Zusammensetzung ohne Tannine eingesetzt.

Die Gefäße wurden mit einem Gummistopfen, durch dessen Bohrung ein Dräger-Diffusionsröhrchen geführt wurde, verschlossen und bei 37°C in einem Brutschrank inkubiert Der freigesetzte Ammoniak wurde in ppm × h gemessen. Die Anfangskeimzahl (gemessen in KBE) des Urins wurde durch das Ausplattieren geeigneter Verdünnungen auf Nährmedienplatten bestimmt. Für die Ammoniakfreisetzung wurde der Mittelwert aus zwei Ansätzen errechnet.

Die geruchsbindenden Eigenschaften der erfindungsgemäßen Pulver B, D, K und L sind der Figur 1 zu entnehmen. Dieser Figur kann entnommen werden, dass insbesondere die Kombination eines nur zu 55 Mol-% teilneutralisierten, wasserabsorbierenden Polymergebildes mit hydrolysierbaren Tanninen zu Produkten mit besonders vorteilhaften, geruchsbindenden Eigenschaften führt.

### Beispiel 5 Einsatz der Tannine in Form von wässrigen Lösungen

Das Beispiel 3 wird wiederholt, wobei die Tannine nicht in pulverform, sondern in Form einer etwa 30 gew.-%igen, wässrigen Lösung auf die Pulver B und D appliziert werden. Die Absorptionseigenschaften der so erhaltenen Pulver M (ausgehend von Pulver B, 70 % neutralisiert) und N (ausgehend von Pulver D, 55 % neutralisiert) sind in der nachfolgenden Tabelle 3 angegeben.

**Tabelle 3**

| | Pulver M | Pulver N |
|---|---|---|
| Tannin | Floctan^{®}1 | Floctan^{®}1 |
| Menge an Tannin, bezogen auf Polymer [Gew.-%] | 0,5 | 0,5 |
| Menge an eingesetzter TanninLösung, bezogen auf 100 g Polymer [g] | 1,7 | 1,7 |
| TB [g/g] | 34,0 | 30,0 |
| AAP₄₈₃₀ Pa (0,7 psi) [g/g] | 20,2 | 19,4 |

Der Tabelle 3 ist zu entnehmen, dass die Tannine auch in Form wässriger Lösungen eingesetzt werden können.

### Beispiel 6 Kombinierter Einsatz von Tanninen und Komplexbildnern

In einem weiteren Versuch wurde Pulver B erneut mit 0,5 Gew.-% Floctan^{®} 1 vermischt, wobei zusätzlich 1.000 ppm Polyethylenglykol (Molekulargewicht = 10.000 g/mol), appliziert in Form einer 4,6 gew.-%igen Lösung, zugesetzt wurde. Dabei wurde Pulver O erhalten.

In einem weiteren Experiment wurde Pulver B mit 0,5 Gew.-% Floctan^{®} 1 sowie mit 0,1 Gew.-% Versenex^{®} (Pentanatriumsalz der Diethylenetriaminpentaessigsäure, appliziert in Form einer 40 gew.-%igen, wässrigen Lösung) vermischt. Dabei wurde Pulver P erhalten.

Von den Pulvern O und P wurden die geruchsbindenden Eigenschaften gemäß der im Beispiel 4 beschriebenen Vorgehensweise bestimmt. Das Ergebnis ist in der Figur 2 dargestellt. Dort ist zu sehen, dass durch den Einsatz eines Komplexbildners (Versenex^{®}) die geruchsbindenden Eigenschaften von mit Tanninen behandelten Superabsorbern noch einmal signifikant verbessert werden können.

### Beispiel 7 Kombinierter Einsatz von Tanninen und Komplexbildnern

In einem weiteren Versuch wurde erneut Pulver B trocken mit 0,5 Gew.-% Floctan^{®} 1 vermischt, Dabei wurde Pulver Q erhalten.

In einem weiteren Experiment wurde Pulver B mit 0,5 Gew.-% Floctan^{®} 1 sowie mit steigenden Mengen an Versenex^{®} (0,1 Gew.-%, 0,5 Gew.-% und 0,75 Gew.-%), appliziert jeweils in Form einer 40 gew.-%igen, wässrigen Lösung) vermischt. Dabei wurden die Pulver R, S und T erhalten.

Von den Pulvern Q, R, S und T wurden die Farbwerte gemäß dem L,a,b-Farbsystem nach Quellung in Fe²⁺-haltiger Lösung, wie im Zusammenhang mit den Testmethoden vorstehend beschrieben, bestimmt. Folgende Werte wurden ermittelt:

**Tabelle 4**

| Pulver | L | a | b |
|---|---|---|---|
| Q | 15,88 | 2,72 | -0,98 |
| R | 27,67 | 7,00 | 1,31 |
| S | 37,44 | 7,54 | 6,30 |
| T | 55,37 | 1,59 | 8,11 |

Wie der Tabelle 4 zu entnehmen ist, führt der kombinierte Einsatz von Tanninen und Komplexbildnern zu einer deutlich verbesserten Farbstabilität der wasserabsorbierenden Zusammensetzung, wenn diese in Kontakt mit Fe²⁺-haltigen Lösung, wie etwa Menstruationsflüssigkeit, tritt.

## Patentansprüche

1. Eine wasserabsorbierende Zusammensetzung, beinhaltend wasserabsorbierende Polymergebilde, auf deren Oberfläche sich mindestens eine Tanninfraktion befindet, die ein gemäß der hierin beschriebenen Testmethode bestimmtes Zahlenmittel des Molekulargewichtes von mindestens 1.000 g/mol besitzt,
oder die ein gemäß der hierin beschriebenen Testmethode bestimmtes Gewichtsmittel des Molekulargewichtes von mindestens 1.000 g/mol besitzt,
wobei die wasserabsorbierende Zusammensetzung das Tannin in einer Menge in einem Bereich von 0,001 bis 10 Gew.-%, bezogen auf das Gesamtgewicht der wasserabsorbierenden Zusammensetzung, beinhaltet.

2. Die wasserabsorbierende Zusammensetzung nach Anspruch 1, wobei die Tanninfraktion ein hydrolysierbares Gallotannin beinhaltet, wobei unter einem hydrolysierbaren Gallotannin ein Tannin zu verstehen ist, das aus einem Polyol als Kern besteht, wobei an die OH-Gruppen des Kernmoleküls Gallsäure über Esterbindungen angebunden sind.

3. Die wasserabsorbierende Zusammensetzung nach einem der vorhergehenden Ansprüche, wobei die Tanninfraktion eine gemäß der hierin beschriebenen Testmethode bestimmte Polydispersität von weniger als 5 aufweist.

4. Die wasserabsorbierende Zusammensetzung nach einem der vorhergehenden Ansprüche, wobei sich das Tannin von der β-1,2,3,4,6-Pentagalloyl-O-D-Glukose ableitet, und wobei an mindestens eine der OH-Gruppen mindestens eines der an den Kohlenhydrat-Kern gebundenen Gallsäure-Moleküle ein weiteres Gallsäure-Molekül über eine Esterbindung gebunden ist, wobei vorzugsweise mindestens 30 Gew.-% der in der wasserabsorbierenden Zusammensetzung enthaltenen Tannine ein mittleres Molekulargewicht von mehr als 940 g/mol aufweisen.

5. Die wasserabsorbierende Zusammensetzung nach einem der vorhergehenden Ansprüche, wobei die wasserabsorbierenden Polymergebilde auf teilneutralisierten, vernetzten Polyacrylaten basieren.

6. Die wasserabsorbierende Zusammensetzung nach einem der vorhergehenden Ansprüche, wobei die wasserabsorbierenden Polymergebilde einen Innenbereich und einen den Innenbereich umgebenden Außenbereich aufweisen, wobei der Außenbereich einen höheren Vernetzungsgrad aufweist als der Innenbereich.

7. Die wasserabsorbierende Zusammensetzung nach einem der vorhergehenden Ansprüche, wobei sich die mindestens eine Tanninfraktion zumindest teilweise in Form von Partikeln auf der Oberfläche der wasserabsorbierenden Polymergebilde befindet.

8. Die wasserabsorbierende Zusammensetzung nach Anspruch 7, wobei mindestens 50 Gew.-% der Partikel der mindestens einen Tanninfraktion eine Größe in einem Bereich von 10 nm bis 300 µm besitzen.

9. Die wasserabsorbierende Zusammensetzung nach Anspruch 7 oder 8, wobei die Partikel der Tanninfraktion über einen Binder auf der Oberfläche der wasserabsorbierenden Polymergebilde immobilisiert sind.

10. Die wasserabsorbierende Zusammensetzung nach einem der vorhergehenden Ansprüche, wobei die mindestens eine Tanninfraktion auf Tanninen basiert, die aus einem Pflanzenextrakt ausgewählt aus der Gruppe bestehend aus *Rhus Semialate-*Extrakt, *Rhus Coriaria*-Extrakt, *Quercus Infectoria*-Extrakt, *Quercus Aegilops-*Extrakt, *Caesalpina Spinosa*-Extrakt, *Caesalpina D. Gyna*-Extrakt, *Valonea*-Extrakt, Kastanienholz-Extrakt und *Terminalia Chebula*-Extrakt oder einer Mischung aus mindestens zwei davon, aufgereinigt worden ist.

11. Die wasserabsorbierende Zusammensetzung nach einem der vorhergehenden Ansprüche, wobei sich auf der Oberfläche der wasserabsorbierenden Polymergebilde zusätzlich zu der Tanninfraktion ein oder mehrere Komplexbildner befinden.

12. Die wasserabsorbierende Zusammensetzung nach Anspruch 11, wobei der Komplexbildner eine Aminopolycarbonsäure ist.

13. Die wasserabsorbierende Zusammensetzung nach Anspruch 11 oder 12, wobei die wasserabsorbierende Zusammensetzung den Komplexbildner in einer Menge in einem Bereich von 0,001 bis 5 Gew.-%, bezogen auf das Gesamtgewicht der wasserabsorbierenden Polymergebilde, beinhaltet.

14. Ein Verfahren zur Herstellung einer wasserabsorbierenden Zusammensetzung, beinhaltend die Verfahrensschritte:
i) Bereitstellen eines wasserabsorbierenden Polymergebildes;
ii) Nachvernetzung des wasserabsorbierenden Polymergebildes;
iii) in Kontakt bringen des wasserabsorbierenden Polymergebildes
a) mit mindestens einer Tanninfraktion, welche ein gemäß der hierin beschriebenen Testmethode bestimmtes Zahlenmittel des Molekulargewichtes von mindestens 1.000 g/mol besitzt, oder
b) mit mindestens einer Tanninfraktion, welche ein gemäß der hierin beschriebenen Testmethode bestimmtes Gewichtsmittel des Molekulargewichtes von mindestens 1.000 g/mol besitzt,
wobei der Verfahrensschritt iii) vor, während oder nach dem Verfahrensschritt ii) durchgeführt werden kann und wobei die mindestens eine Tanninfraktion in einer Menge in einem Bereich von 0,001 bis 10 Gew.-%, bezogen auf das Gesamtgewicht aus wasserabsorbierenden Polymergebilden, eingesetzt wird.

15. Das Verfahren nach Anspruch 14, wobei die im Verfahrensschritt i) bereitgestellten, wasserabsorbierenden Polymergebilde auf teilneutralisierten, vernetzten Polyacrylaten basieren.

16. Das Verfahren nach einem der Ansprüche 14 und 15, wobei die mindestens eine Tanninfraktion im Verfahrensschritt iii) in Form von Partikeln eingesetzt wird.

17. Das Verfahren nach Anspruch 16, wobei mindestens 50 Gew.-% der Partikel der mindestens einen Tanninfraktion eine Größe in einem Bereich von 10 nm bis 300 µm besitzen.

18. Das Verfahren nach einem der Ansprüche 14 bis 17, wobei die mindestens eine Tanninfraktion, welche im Verfahrensschritt iii) eingesetzt wird, mindestens eine der folgenden Eigenschaften aufweist:
A) eine Dichte in einem Bereich von 0,2 bis 0,6 g/cm³;
B) einen pH-Wert als 1 gew.-%iger Lösung in Wasser bei 20°C in einem Bereich von 2 bis 5;
C) einen Gehalt an freier Gallsäure von weniger als 0,5 Gew.-%, bezogen auf das Gesamtgewicht der Tanninfraktion;
D) eine Reinheit, angegeben als Tanninanteil, von mindestens 90 Gew.-%, bezogen auf das Gesamtgewicht der Tanninfraktion.

19. Das Verfahren nach einem der Ansprüche 14 bis 18, wobei die mindestens eine Tanninfraktion, die im Verfahrensschritt iii) eingesetzt wird, auf Tanninen basiert, die aus einem Pflanzenextrakt ausgewählt aus der Gruppe bestehend aus *Rhus Semialata-*Extrakt, *Rhus Coriaria-Extrakt, Quercus Infectoria*-Extrakt, *Quercus Aegilops-*Extrakt, *Caesalpina Spinosa*-Extrakt, *Caesalpina D. Gyna*-Extrakt, *Valonea*-Extrakt, Kastanienholz-Extrakt und Terminalia Chebula-Extrakt oder einer Mischung aus mindestens zwei davon, aufgereinigt worden ist.

20. Das Verfahren nach einem der Ansprüche 14 bis 19, beinhaltend den weiteren Verfahrensschritt des
iv) in Kontakt bringens der wasserabsorbierenden Polymergebilde mit einem oder mehreren Komplexbildner(n).

21. Das Verfahren nach Anspruch 20, wobei der Komplexbildner eine Aminopolycarbonsäure ist.

22. Das Verfahren nach Anspruch 20 oder 21, wobei der Komplexbildner in einer Menge in einem Bereich von 0,001 bis 5 Gew.-%, bezogen auf das Gesamtgewicht der wasserabsorbierenden Polymergebilde, eingesetzt wird.

23. Wasserabsorbierende Zusammensetzung, erhältlich nach einem Verfahren nach einem der Ansprüche 14 bis 21.

24. Wasserabsorbierende Zusammensetzung nach einem der Ansprüche 1 bis 13 oder 23, wobei die wasserabsorbierende Zusammensetzung mindestens eine der folgenden Eigenschaften aufweist:
(β1) eine nach ERT 441.2-02 bestimmte Retention von mindestens 20g/g;
(β2) eine (im Falle von Partikeln für die gesamte Partikelgrößenfraktion) bestimmte Absorption gegen einen Druck von 0,7 psi (50 g/cm²) nach ERT 442.2-02 von mindestens 15 g/g;
(β3) einen Neutralisationsgrad von höchstens 78 Mol-%.

25. Verwendung der wasserabsorbierenden Zusammensetzung nach einem der Ansprüche 1 bis 13, 23 oder 24 in Schäumen, Formkörpern, Fasern, Folien, Filmen, Kabeln, Dichtungsmaterialien, flüssigkeitsaufnehmenden Hygieneartikeln, Trägern für pflanzen- und pilzwachstumregulierenden Mitteln, Verpackungsmaterialien, Bodenzusätzen, zur kontrollierten Freisetzung von Wirkstoffen oder in Baustoffen.

## Claims

1. Water-absorbing composition comprising water-absorbing polymer structures, on the surface of which is at least one tannin fraction which has a number-average molecular weight, determined in accordance with the test method described herein, of at least 1,000 g/mol or which has a weight-average molecular weight, determined in accordance with the test method described herein, of at least 1,000 g/mol,
wherein the water-absorbing composition comprises the tannin in an amount in a range of from 0.001 to 10 wt. %, based on the total weight of the water-absorbing composition.

2. Water-absorbing composition according to Claim 1, wherein the tannin fraction comprises a hydrolyzable gallotannin, wherein a hydrolyzable gallotannin is to be understood as meaning a tannin consisting of a polyol as a core, wherein gallic acid is bonded to the OH groups of the core molecule via ester bonds.

3. Water-absorbing composition according to any of the preceding claims, wherein the tannin fraction has a polydispersity, determined in accordance with the test method described herein, of less than 5.

4. Water-absorbing composition according to any of the preceding claims, wherein the tannin is derived from β-1,2,3,4,6-pentagalloyl-O-D-glucose, and wherein to at least one of the OH groups of at least one of the gallic acid molecules bonded to the carbohydrate core there is bonded a further gallic acid molecule via an ester bond, wherein preferably at least 30 wt. % of the tannins contained in the water-absorbing composition have an average molecular weight of more than 940 g/mol.

5. Water-absorbing composition according to any of the preceding claims, wherein the water-absorbing polymer structures are based on partly neutralized, crosslinked polyacrylates.

6. Water-absorbing composition according to any of the preceding claims, wherein the water-absorbing polymer structures have an inner region and an outer region surrounding the inner region, wherein the outer region has a higher degree of crosslinking than the inner region.

7. Water-absorbing composition according to any of the preceding claims, wherein the at least one tannin fraction is at least partly in the form of particles on the surface of the water-absorbing polymer structures.

8. Water-absorbing composition according to Claim 7, wherein at least 50 wt. % of the particles of the at least one tannin fraction have a size in a range of from 10 nm to 300 µm.

9. Water-absorbing composition according to Claim 7 or 8, wherein the particles of the tannin fraction are immobilized on the surface of the water-absorbing polymer structures via a binder.

10. Water-absorbing composition according to any of the preceding claims, wherein the at least one tannin fraction is based on tannins which have been purified from a plant extract chosen from the group consisting of Rhus semialata extract, Rhus coriaria extract, Quercus infectoria extract, Quercus aegilops extract, Caesalpina spinosa extract, Caesalpina d. gyna extract, Valonea extract, Chestnut wood extract and Terminalia chebula extract or a mixture of at least two of these.

11. Water-absorbing composition according to any of the preceding claims, wherein one or more complex-formers are present on the surface of the water-absorbing polymer structures in addition to the tannin fraction.

12. Water-absorbing composition according to Claim 11, wherein the complex-former is an aminopolycarboxylic acid.

13. Water-absorbing composition according to Claim 11 or 12, wherein the water-absorbing composition contains the complex-former in an amount in a range of from 0.001 to 5 wt. %, based on the total weight of the water-absorbing polymer structures.

14. Process for the preparation of a water-absorbing composition comprising the process steps:
i) provision of a water-absorbing polymer structure;
ii) post-crosslinking of the water-absorbing polymer structure;
iii) bringing the water-absorbing polymer structure into contact
a) with at least one tannin fraction which has a number-average molecular weight, determined in accordance with the test method described herein, of at least 1,000 g/mol, or
b) with at least one tannin fraction which has a weight-average molecular weight, determined in accordance with the test method described herein, of at least 1,000 g/mol,
wherein process step iii) can be carried out before, during or after process step ii) and wherein the at least one tannin fraction is employed in an amount in a range of from 0.001 to 10 wt. %, based on the total weight of water-absorbing polymer structures.

15. Process according to Claim 14, wherein the water-absorbing polymer structures provided in process step i) are based on partly neutralized crosslinked polyacrylates.

16. Process according to either of Claims 14 and 15, wherein the at least one tannin fraction is employed in process step iii) in the form of particles.

17. Process according to Claim 16, wherein at least 50 wt. % of the particles of the at least one tannin fraction have a size in a range of from 10 nm to 300 µm.

18. Process according to any of Claims 14 to 17, wherein the at least one tannin fraction which is employed in process step iii) has at least one of the following properties:
A) a density in a range of from 0.2 to 0.6 g/cm³;
B) a pH as a 1 wt. % solution in water at 20°C in a range of from 2 to 5;
C) a content of free gallic acid of less than 0.5 wt. %, based on the total weight of the tannin fraction;
D) a purity, stated as the tannin content, of at least 90 wt. %, based on the total weight of the tannin fraction.

19. Process according to any of Claims 14 to 18, wherein the at least one tannin fraction which is employed in process step iii) is based on tannins which have been purified from a plant extract chosen from the group consisting of Rhus semialata extract, Rhus coriaria extract, Quercus infectoria extract, Quercus aegilops extract, Caesalpina spinosa extract, Caesalpina d. gyna extract, Valonea extract, Chestnut wood extract and Terminalia chebula extract or a mixture of at least two of these.

20. Process according to any of Claims 14 to 19, including the further process step of
iv) bringing the water-absorbing polymer structures into contact with one or more complex former(s).

21. Process according to Claim 20, wherein the complex-former is an aminopolycarboxylic acid.

22. Process according to Claim 20 or 21, wherein the complex-former is employed in an amount in a range of from 0.001 to 5 wt. %, based on the total weight of the water-absorbing polymer structures.

23. Water-absorbing composition obtainable by a process according to any of Claims 14 to 21.

24. Water-absorbing composition according to any of Claims 1 to 13 or 23, wherein the water-absorbing composition has at least one of the following properties:
(β1) a retention, determined in accordance with ERT 441.2-02, of at least 20 g/g;
(β2) an absorption, determined in accordance with ERT 441.2-02, (in the case of particles for the total particle size fraction) against a pressure of 0.7 psi (50 g/cm²) of at least 15 g/g;
(β3) a degree of neutralization of not more than 78 mol %.

25. Use of the water-absorbing composition according to any of Claims 1 to 13, 23 or 24 in foams, shaped articles, fibres, foils, films, cables, sealing materials, liquid-absorbing hygiene articles, carriers for plant or fungal growth-regulating agents, packaging materials, soil additives, for controlled release of active compounds or in building materials.

## Revendications

1. Composition absorbant l'eau, comprenant des structures polymères absorbant l'eau sur la surface desquelles se trouvent au moins une fraction de tannin qui possède une moyenne numérique du poids moléculaire, déterminée selon la méthode de test décrite dans la description, d'au moins 1000 g/mole, ou qui possède une moyenne pondérale du poids moléculaire, déterminée selon la méthode de test décrite dans la description, d'au moins 1000 g/mole, la composition absorbant l'eau comprenant le tannin en une quantité dans la plage de 0,001 à 10% en poids, par rapport au poids total de la composition absorbant l'eau.

2. Composition absorbant l'eau selon la revendication 1, la fraction de tannin comprenant un gallotannin hydrolysable, un gallotannin hydrolysable désignant un tannin qui est constitué par un polyol comme noyau, de l'acide gallique étant lié par l'intermédiaire de liaisons ester aux groupes OH de la molécule de noyau.

3. Composition absorbant l'eau selon l'une quelconque des revendications précédentes, la fraction de tannin présentant une polydispersité, déterminée selon la méthode de test décrite dans la description, inférieure à 5.

4. Composition absorbant l'eau selon l'une quelconque des revendications précédentes, le tannin étant dérivé du β-1,2,3,4,6-pentagalloyl-O-D-glucose et une autre molécule d'acide gallique étant liée par l'intermédiaire d'une liaison ester à au moins l'un des groupes OH d'au moins une des molécules d'acide gallique liées au noyau glucidique, au moins 30% en poids des tannins contenus dans la composition absorbant l'eau présentant de préférence un poids moléculaire moyen supérieur à 940 g/mole.

5. Composition absorbant l'eau selon l'une quelconque des revendications précédentes, les structures polymères absorbant l'eau étant basées sur des polyacrylates partiellement neutralisés, réticulés.

6. Composition absorbant l'eau selon l'une quelconque des revendications précédentes, les structures polymères absorbant l'eau présentant une zone interne et une zone externe entourant la zone interne, la zone externe présentant un degré de réticulation supérieur à celui de la zone interne.

7. Composition absorbant l'eau selon l'une quelconque des revendications précédentes, ladite au moins une fraction de tannin se trouvant au moins partiellement sous forme de particules sur la surface des structures polymères absorbant l'eau.

8. Composition absorbant l'eau selon la revendication 7, au moins 50% des particules de ladite au moins une fraction de tannin possédant une grosseur dans la plage de 10 nm à 300 µm.

9. Composition absorbant l'eau selon la revendication 7 ou 8, les particules de la fraction de tannin étant immobilisées par l'intermédiaire d'un liant sur la surface des structures polymères absorbant l'eau.

10. Composition absorbant l'eau selon l'une quelconque des revendications précédentes, ladite au moins une fraction de tannin étant à base de tannins qui ont été purifiés à partir d'un extrait végétal choisi dans le groupe constitué par un extrait de Rhus Semialata, un extrait de Rhus Coriaria, un extrait de Quercus Infectoria, un extrait de Quercus Aegilops, un extrait de Caesalpinia Spinosa, un extrait de Caesalpinia D. Gyna, un extrait de Valonea, un extrait de bois de châtaignier et un extrait de Terminalia Chebula ou un mélange d'au moins deux de ceux-ci.

11. Composition absorbant l'eau selon l'une quelconque des revendications précédentes, un ou plusieurs complexants se trouvant en plus de la fraction de tannin sur la surface des structures polymères absorbant l'eau.

12. Composition absorbant l'eau selon la revendication 11, le complexant étant un acide aminopolycarboxylique.

13. Composition absorbant l'eau selon la revendication 11 ou 12, la composition absorbant l'eau comprenant le complexant en une quantité dans la plage de 0,001 à 5% en poids par rapport au poids total des structures polymères absorbant l'eau.

14. Procédé pour la préparation d'une composition absorbant l'eau, comprenant les étapes de procédé de :
i) préparation d'une structure polymère absorbant l'eau ;
ii) postréticulation de la structure polymère absorbant l'eau ;
iii) mise en contact de la structure polymère absorbant l'eau
a) avec au moins une fraction de tannin qui possède une moyenne numérique du poids moléculaire, déterminée selon la méthode de test décrite dans la description, d'au moins 1000 g/mole ou
b) avec au moins une fraction de tannin qui possède une moyenne pondérale du poids moléculaire, déterminée selon la méthode de test décrite dans la description, d'au moins 1000 g/mole,
l'étape de procédé iii) pouvant être réalisée avant, pendant ou après l'étape de procédé ii) et ladite au moins une fraction de tannin étant utilisée en une quantité dans la plage de 0,001 à 10% en poids, par rapport au poids total des structures polymères absorbant l'eau.

15. Procédé selon la revendication 14, les structures polymères absorbant l'eau, préparées dans l'étape de procédé i), étant basées sur des polyacrylates partiellement neutralisés, réticulés.

16. Procédé selon l'une quelconque des revendications 14 et 15, ladite au moins une fraction de tannin étant utilisée sous forme de particules dans l'étape de procédé iii).

17. Procédé selon la revendication 16, au moins 50% des particules de ladite au moins une fraction de tannin possédant une grosseur dans la plage de 10 nm à 300 µm.

18. Procédé selon l'une quelconque des revendications 14 à 17, ladite au moins une fraction de tannin, qui est utilisée dans l'étape de procédé iii), présentant au moins l'une des propriétés suivantes :
A) une masse volumique dans la plage de 0,2 à 0,6 g/cm³ ;
B) une valeur de pH, sous forme de solution à 1% en poids dans l'eau à 20°C, dans la plage de 2 à 5 ;
C) une teneur en acide gallique libre inférieure à 0,5% en poids par rapport au poids total de la fraction de tannin ;
D) une pureté, indiquée sous forme de proportion de tannin, d'au moins 90% en poids par rapport au poids total de la fraction de tannin.

19. Procédé selon l'une quelconque des revendications 14 à 18, ladite au moins une fraction de tannin, qui est utilisée dans l'étape de procédé iii), étant à base de tannins qui ont été purifiés à partir d'un extrait végétal choisi dans le groupe constitué par un extrait de Rhus Semialata, un extrait de Rhus Coriaria, un extrait de Quercus Infectoria, un extrait de Quercus Aegilops, un extrait de Caesalpinia Spinosa, un extrait de Caesalpinia D. Gyna, un extrait de Valonea, un extrait de bois de châtaignier et un extrait de Terminalia Chebula ou un mélange d'au moins deux de ceux-ci.

20. Procédé selon l'une quelconque des revendications 14 à 19, comprenant l'étape de procédé supplémentaire de
iv) mise en contact des structures polymères absorbant l'eau avec un ou plusieurs complexants.

21. Procédé selon la revendication 20, le complexant étant un acide aminopolycarboxylique.

22. Procédé selon la revendication 20 ou 21, le complexant étant utilisé en une quantité dans la plage de 0,001 à 5% en poids, par rapport au poids total de la structure polymère absorbant l'eau.

23. Composition absorbant l'eau, pouvant être obtenue selon un procédé selon l'une quelconque des revendications 14 à 21.

24. Composition absorbant l'eau selon l'une quelconque des revendications 1 à 13 ou 23, la composition absorbant l'eau présentant au moins l'une des propriétés suivantes :
(β1) une rétention, déterminée selon la norme ERT 441.2-02, d'au moins 20 g/g ;
(β2) une absorption déterminée (dans le cas de particules pour toute la fraction granulométrie) par rapport à une pression de 0,7 psi (50 g/cm²) selon la norme ERT 442.2-02 d'au moins 15 g/g ;
(β3) un degré de neutralisation d'au plus 78% en mole.

25. Utilisation de la composition absorbant l'eau selon l'une quelconque des revendications 1 à 13, 23 ou 24 dans des mousses, des corps façonnés, des fibres, des feuilles, des films, des câbles, des matériaux d'étanchéité, des articles hygiéniques absorbant les liquides, des supports pour les agents régulant la croissance des plantes et des champignons, des matériaux d'emballage, des amendements de sol, pour la libération contrôlée de substances actives ou dans des matériaux de construction.
